# EUROPEAN PATENT SPECIFICATION

(11) **EP 4 126 822 B1**
(45) Date of publication and mention of the grant of the patent: **12.08.2026**
(21) Application number: 21774577.7
(22) Date of filing: 24.03.2021
(51) Int. Cl.: A61K 9/16, A61K 9/20, A61K 31/415, A61K 31/4439, A61K 31/454, A61P 3/04, C07D 231/06, C07D 401/12

(54) **SOLID DISPERSIONS OF AMORPHOUS 3, 4-DIPHENYL-4, 5-DIHYDRO- 1H-PYRAZOLE DERIVATIVES, COMPOSITIONS COMPRISING THEM AND USES THEREOF AS CANNABINOID CB1 RECEPTOR INHIBITORS**
FESTE DISPERSIONEN VON AMORPHEN 3,4-DIPHENYL-4,5-DIHYDRO-1H-PYRAZOL-DERIVATEN, ZUSAMMENSETZUNGEN DAMIT UND VERWENDUNGEN DAVON ALS CANNABINOID-CB1-REZEPTORHEMMER
DISPERSIONS SOLIDES DE DÉRIVÉS DE 3,4-DIPHÉNYL-4,5-DIHYDRO-1H-PYRAZOLE AMORPHES, COMPOSITIONS LES COMPRENANT ET LEURS UTILISATIONS EN TANT QU'INHIBITEURS DU RÉCEPTEUR CANNABINOÏDE CB1

(30) Priority: 24.03.2020 US 202062993775 P
(43) Date of publication of application: 08.02.2023
(73) Proprietor: Novo Nordisk A/S, 2880 Bagsværd (DK)
(72) Inventor: RAVENELLE, Francois, Montreal, Québec H2G 2K8 (CA); VALDES, Norelys, Montreal, Québec H1J 1Y4 (CA); BETANCOURT, Aimesther O., Montreal, Québec H1E 3W5 (CA); GOSSELIN, Patrick, Laval, Québec H7X 4H1 (CA)
(86) International application number: PCT/CA2021/050385
(87) International publication number: WO 2021/189141

(56) References cited:
- WO-A1-2014/078309
- WO-A1-2014/078309
- WO-A1-2016/011525
- WO-A1-2016/196646
- WO-A1-2020/236411
- WAYNE SINCLAIR: "Physical stability and recrystallization kinetics of amorphous ibipinabant drug product by fourier transform raman spectroscopy", JOURNAL OF PHARMACEUTICAL SCIENCES, vol. 100, no. 11, 1 November 2011 (2011-11-01), US, pages 4687 - 4699, XP093164825, ISSN: 0022-3549, DOI: 10.1002/jps.22658
- IYER MALLIGA R. ET AL: "Synthesis of 13 C 6 -labeled, dual-target inhibitor of cannabinoid-1 receptor (CB 1 R) and inducible nitric oxide synthase (iNOS)", vol. 61, no. 10, 25 June 2018 (2018-06-25), GB, pages 773 - 779, XP055898213, ISSN: 0362-4803, Retrieved from the Internet <URL:https://api.wiley.com/onlinelibrary/tdm/v1/articles/10.1002%2Fjlcr.3639> DOI: 10.1002/jlcr.3639
- SAMEER SINGH: "A review on solid dispersion", vol. 2, no. 9, 1 November 2011 (2011-11-01), IN, pages 1078 - 1095, XP093164786, ISSN: 0976-7126, Retrieved from the Internet <URL:https://www.ijplsjournal.com/issues%20PDF%20files/sep2011/11.pdf>
- L.S. RANZANI: "Enhanced in vivo absorption of CB-1 antagonist in rats via solid solutions prepared by hot-melt extrusion", vol. 37, no. 6, 13 January 2011 (2011-01-13), US, pages 694 - 701, XP093164804, ISSN: 0363-9045, Retrieved from the Internet <URL:https://www.tandfonline.com/doi/epdf/10.3109/03639045.2010.535822> DOI: 10.3109/03639045.2010.535822
- MICHAEL M. LEANE: "Formulation and process design for a solid dosage form containing a spray-dried amorphous dispersion of ibipinabant", vol. 18, no. 2, 23 January 2012 (2012-01-23), US, pages 359 - 366, XP093165249, ISSN: 1083-7450, Retrieved from the Internet <URL:https://www.tandfonline.com/doi/full/10.3109/10837450.2011.619544> DOI: 10.3109/10837450.2011.619544
- MALLIGA R. IYER, CINAR RESAT, KATZ ALEXIS, GAO MICHAEL, ERDELYI KATALIN, JOURDAN TONY, COFFEY NATHAN J., PACHER PAL, KUNOS GEORGE: "Design, Synthesis, and Biological Evaluation of Novel, Non-Brain-Penetrant, Hybrid Cannabinoid CB 1 R Inverse Agonist/Inducible Nitric Oxide Synthase (iNOS) Inhibitors for the Treatment of Liver Fibrosis", JOURNAL OF MEDICINAL CHEMISTRY, AMERICAN CHEMICAL SOCIETY, US, vol. 60, no. 3, 9 February 2017 (2017-02-09), US , pages 1126 - 1141, XP055368197, ISSN: 0022-2623, DOI: 10.1021/acs.jmedchem.6b01504
- IYER MALLIGA R., CINAR RESAT, COFFEY NATHAN J., KUNOS GEORGE: "Synthesis of 13 C 6 -labeled, dual-target inhibitor of cannabinoid-1 receptor (CB 1 R) and inducible nitric oxide synthase (iNOS)", JOURNAL OF LABELLED COMPOUNDS AND RADIOPHARMACEUTICALS, JOHN WILEY & SONS LTD., GB, vol. 61, no. 10, 1 August 2018 (2018-08-01), GB , pages 773 - 779, XP055898213, ISSN: 0362-4803, DOI: 10.1002/jlcr.3639
- FELTON LINDA: "Remington Essentials of Pharmaceutics", PHARMACEUTICAL PRESS, REMINGTON, XP055898216, Retrieved from the Internet <URL:https://health.sbmu.ac.ir/uploads/Remington_Essentials_of_Pharmaceutics_-_Felton,_Linda.pdf> [retrieved on 20220307], DOI: 9780857111050

## Description

### RELATED APPLICATION

The present application claims priority under applicable law to United States provisional application No. 62/993,775 filed on March 24, 2020.

### TECHNICAL FIELD

This disclosure generally relates to amorphous compounds, solid dispersions of amorphous compounds, pharmaceutical compositions comprising the same and their use in the treatment and prevention of diseases and disorders.

### BACKGROUND

It is generally known that activation of the cannabinoid CB₁ receptor increases appetite, increases the biosynthesis and storage of lipids, inhibits the actions of insulin and leptin, and promotes inflammation and fibrosis. Research was thus focused on developing CB₁ receptor inhibitors for the potential treatment of obesity and the metabolic disorder associated therewith, referred to as metabolic syndrome. Rimonabant was shown effective in treating metabolic syndrome but caused neuropsychiatric (i.e. CNS-related) side effects, which resulted in its withdrawal from the market. Compounds preferentially targeting the CB₁ receptor in peripheral tissues (e.g. adipose tissue, liver, muscle, lung, kidney, macrophages, pancreatic beta cells and gastrointestinal tract), while not interacting with CB₁ receptors in brain tissue, thereby avoiding or reducing CNS-related side effects, were disclosed by George Kunos et al. in U.S. Patent 9,765,031. WO 2014/078309 A1 discloses pharmaceutical compositions comprising a cannabinoid-1 receptor antagonist and and at least one pharmaceutically acceptable additive, useful for treating e.g. obesity, diabetes, non-alcoholic and alcoholic fatty liver disease, diabetic nephropathy, or gout.

### SUMMARY

According to one aspect, the present technology relates to a solid dispersion comprising a compound dispersed in a solid matrix comprising a pharmaceutically acceptable polymer having a glass transition temperature of at least 50°C, wherein the compound is selected from: 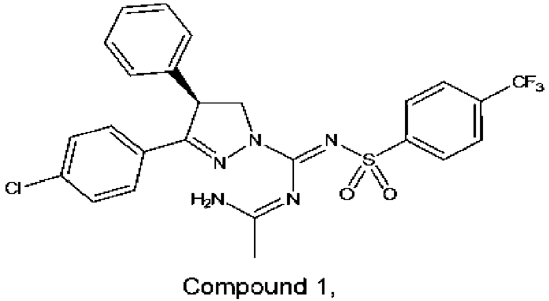 or 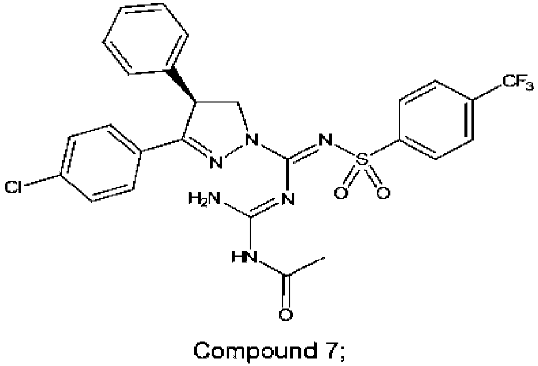
or a tautomer or a pharmaceutically acceptable salt thereof;
wherein the compound is in a substantially amorphous form;
wherein the polymer:compound weight ratio is within the range of 1:1 to 6:1, or preferably 1:1 to 4:1, or preferably 2:1 to 5:1, or preferably 3:1 to 5:1, or preferably 1:1 to 3:1, and
wherein the compound concentration in the solid dispersion is within the range of 15% to 60% by weight preferably 18% to 40% by weight, or preferably within the range of 20% to 40% by weight, or preferably within the range of 30% to 50% by weight.

In a further embodiment of the above solid dispersion, the polymer has a glass transition temperature of at least 80°C, or at least 100°C, or at least 120°C, or at least 140°C, or between 50°C and 200°C, or between 80°C and 200°C, or between 100°C and 180°C. For instance, such a polymer is a polyvinylpyrrolidone or a copolymer thereof, such as a polyvinylpyrrolidone having an average molecular weight of between 5,000 and 100,000. In another example, the polymer is cellulose or a cellulose derivative like an esterified hydroxyalkyl methylcellulose, e.g. hydroxypropyl methylcellulose acetate succinate. In yet other examples, the polymer is a polyethylene glycol, polylactic acid or polymethacrylate.

In some embodiments, the polymer:compound weight ratio is within the range of 1:1 to 6:1, or between 1:1 and 4:1, or between 2:1 and 5:1, or between 3:1 and 5:1, or between 1:1 and 3:1.

In other embodiments, the solid dispersion further comprises a pharmaceutically acceptable surfactant. For instance, the pharmaceutically acceptable surfactant comprises at least one surfactant selected from long chain alkyl or alkenyl sulfate salts (e.g. sulfates of C₈ to C₂₀ alcohols, such as sodium lauryl sulfate, sodium laureth sulfate, ammonium lauryl sulfate, sodium pareth sulfate, and the like); alkyl sulfonate salts (e.g. perfluorooctanesulfonate, perfluorobutanesulfonate, docusate, and the like); sorbitan long chain carboxylic acid esters (e.g. C₈ to C₂₀ carboxylic acids such as oleate, stearate, laurate, and the like); pegylated sorbitan long chain carboxylic acid esters (e.g. Tween 20, Tween 40, Tween 60, Tween 80); polyethylene-polypropylene glycol block copolymers (e.g. poloxamers); pegylated or non-pegylated mono, di, and tri glyceride long chain carboxylic acid esters (e.g. PEG-4, -6 or -8, C₈ to C₂₀ alkyl carboxylic acid triglycerides); polyethylene and/or polypropylene glycol alkoxylates; alkylphenol alkoxylates; alkylphenol derivatives of polyethylene and/or polypropylene glycols (e.g. Triton X-100); and sucrose long chain carboxylic acid esters. In one embodiment, the pharmaceutically acceptable surfactant is a long chain alkyl sulfate (e.g. sodium lauryl sulfate, sodium laureth sulfate, ammonium lauryl sulfate, sodium pareth sulfate, and the like). According to some embodiments, the polymer:surfactant weight ratio is within the range of 5:1 to 20:1, or of 10:1 to 15:1.

According to a preferred embodiment, the compound comprises less than of less than 5% of crystalline form, or less than 2% of crystalline form, or less than 1% of crystalline form, or even less than 0.5% of crystalline form. In some examples, the solid dispersion described herein and according to any of the aforementioned embodiments is in powder form. Alternatively, the solid dispersion described herein and according to any of the aforementioned embodiments is in particulate form.

According to another aspect, the present technology relates to a process for the preparation of a solid dispersion as defined herein, comprising a step of mixing the compound and the polymer. In one embodiment, the mixing step comprises the steps of: (a) dissolving the compound and polymer in a solvent; and (b) drying the mixture obtained in (a). For instance, the drying step is carried out by spray drying. In another embodiment, the mixing step is carried out by rapid acoustic mixing, extrusion, planetary mixing and ball milling.

According to a further aspect, the present technology relates to a solid oral pharmaceutical composition comprising a solid dispersion as defined herein. In one embodiment, the solid oral pharmaceutical composition further comprises a pharmaceutically acceptable carrier, diluent or excipient. For example, the said carrier, diluent or excipient is a binder such as a binder selected from cellulose-based substances such as microcrystalline cellulose and carboxymethylcellulose, and other binders like gum acacia, gelatin, corn starch, gum tragacanth, sodium alginate, lactose, sorbitol, mannitol, dextrose, kaolin, cellulose, calcium carbonate, calcium silicate, dicalcium phosphate, and polyethylene glycol (PEG), e.g. microcrystalline cellulose.

In one embodiment, the compound is present in the composition at a concentration of between 5 wt.% and 50 wt.%, or between 10 wt.% and 40 wt.%. In another embodiment, the solid oral pharmaceutical composition is in the form of a tablet or capsule. In a further embodiment, the solid oral pharmaceutical composition further comprises a coating. In yet another embodiment, the solid oral pharmaceutical composition is in unit dosage form comprising the compound in an amount within the range of 20 to 200 mg per dose.

According to yet another aspect, the present technology relates to the use of a solid dispersion as defined herein or a solid oral pharmaceutical composition as defined herein for the treatment of a disease or disorder selected from obesity(type I or II), non-alcoholic and alcoholic fatty liver disease (a risk factor for insulin resistance), a co-morbidity of obesity, a co-morbidity of diabetes, Prader-Willi Syndrome (PWS), Pro-opiomelanocortin (POMC) deficiency obesity, LepR deficiency obesity, POMC heterozygous deficiency obesity, POMC epigenetic disorders, Bardet-Biedl syndrome, Alström syndrome, dyslipidemia predisposing to arteriosclerotic heart disease, diabetic nephropathy, fibrosis and fibrotic diseases such as Idiopathic Pulmonary Fibrosis (IPF) and Hermansky-Pudlak Syndrome pulmonary fibrosis (HPS-PF), and gout. In one embodiment, the co-morbidity of obesity is selected from metabolic syndrome, dementia, heart disease, hypertension, gallbladder disease, gastrointestinal disorders, menstrual irregularities, degenerative arthritis, venous statis ulcer, pulmonary hypoventilation syndrome, sleep apnea, snoring, coronary artery disease, arterial sclerotic disease, pseudotumor cerebri, osteoarthritis, high cholesterol, and increased incidence of malignancies of the liver, ovaries, cervix, uterus, breasts, prostate, or gallbladder. In another embodiment, the co-morbidity of diabetes (e.g. type I) is selected from diabetic nephropathy, chronic kidney disease, diabetic retinopathy, and peripheral and autonomic neuropathy. In a further embodiment, the disease or disorder is selected from diabetes (type 1 or 2), obesity, and non-alcoholic fatty liver disease (e.g. non-alcoholic steatohepatitis).

Other aspects of the present technology relates to a method for the treatment of a disease or disorder selected from obesity, diabetes(type I or II), non-alcoholic and alcoholic fatty liver disease (a risk factor for insulin resistance), a co-morbidity of obesity, a co-morbidity of diabetes, Prader-Willi Syndrome (PWS), Pro-opiomelanocortin (POMC) deficiency obesity, LepR deficiency obesity, POMC heterozygous deficiency obesity, POMC epigenetic disorders, Bardet-Biedl syndrome, Alström syndrome, dyslipidemia predisposing to arteriosclerotic heart disease, diabetic nephropathy, fibrosis and fibrotic diseases such as Idiopathic Pulmonary Fibrosis (IPF) and Hermansky-Pudlak Syndrome pulmonary fibrosis (HPS-PF), and gout, comprising administering a solid dispersion as defined herein or a solid oral pharmaceutical composition as defined herein to a subject in need thereof. In one embodiment, the co-morbidity of obesity is selected from metabolic syndrome, dementia, heart disease, hypertension, gallbladder disease, gastrointestinal disorders, menstrual irregularities, degenerative arthritis, venous statis ulcer, pulmonary hypoventilation syndrome, sleep apnea, snoring, coronary artery disease, arterial sclerotic disease, pseudotumor cerebri, osteoarthritis, high cholesterol, and increased incidence of malignancies of the liver, ovaries, cervix, uterus, breasts, prostate, or gallbladder. In another embodiment, the co-morbidity of diabetes (e.g. type I) is selected from diabetic nephropathy, chronic kidney disease, diabetic retinopathy, and peripheral and autonomic neuropathy. In a further embodiment, the disease or disorder is selected from diabetes (type 1 or 2), obesity, and non-alcoholic fatty liver disease (e.g. non-alcoholic steatohepatitis).

Additional objects and features of the present compound, compositions, methods and uses will become more apparent upon reading of the following non-restrictive description of exemplary embodiments and examples section, which should not be interpreted as limiting the scope of the invention.

### BRIEF DESCRIPTION OF THE FIGURES

Figure 1 shows x-ray powder diffraction patterns of formulations F1, compared to F-MP, F-SEDDS, and Compound 1 as described in Example 2.
Figure 2 shows x-ray powder diffraction patterns of formulations F2, F3, F4, F5 and Compound 1 as described in Example 2.

### DETAILED DESCRIPTION

All technical and scientific terms and expressions used herein have the same definitions as those commonly understood by a person skilled in the art to which the present technology pertains. The definition of some terms and expressions used is nevertheless provided below. To the extent the definitions of terms in the publications, patents, and patent applications mentioned herein are contrary to the definitions set forth in this specification, the definitions in this specification will control. The section headings used herein are for organizational purposes only and are not to be construed as limiting the subject matter disclosed.

Chemical structures described herein are drawn according to conventional standards. Also, when an atom, such as a carbon atom, as drawn seems to include an incomplete valency, then the valency is assumed to be satisfied by one or more hydrogen atoms even though these are not necessarily explicitly drawn. Hydrogen atoms should be inferred to be part of the compound.

The terminology used herein is for the purpose of describing particular embodiments only and is not intended to be limiting. It should be noted that, the singular forms "a", "an", and "the" include plural forms as well, unless the content clearly dictates otherwise. Thus, for example, reference to a composition containing "a compound" also contemplates a mixture of two or more compounds. It should also be noted that the term "or" is generally employed in its sense including "and/or" unless the context clearly dictates otherwise. Furthermore, to the extent that the terms "including", "includes", "having", "has", "with", or variants thereof are used in either the detailed description and/or the claims, such terms are intended to be inclusive in a manner similar to the term "comprising".

The term "about" or "approximately" means within an acceptable error range for the particular value as determined by one of ordinary skill in the art, which will depend in part on how the value is measured or determined, i.e., the limitations of the measurement system. For example, "about" can mean within 1 or more than 1 standard deviation, per the practice in the art. Alternatively, "about" can mean a range of up to 20%, preferably up to 10%, more preferably up to 5%, and more preferably still up to 1% of a given value. Alternatively, particularly with respect to biological systems or processes, the term can mean within an order of magnitude, preferably within 5-fold, and more preferably within 2-fold, of a value. Where particular values are described in the application and claims, unless otherwise stated the term "about" meaning within an acceptable error range for the particular value should be assumed.

As used herein, the terms "compounds", "active ingredient", and equivalent expressions refer to compounds described in the present application and in U.S. Patent No. 9,765,031, e.g. those encompassed by structural Formulae I and I(a), optionally with reference to any of the applicable embodiments, and also includes exemplary compounds, such as Compounds 1 to 3, as well as their pharmaceutically acceptable salts, tautomeric forms, solvates, esters, and prodrugs when applicable. When a zwitterionic form is possible, the compound may be drawn as its neutral form for practical purposes, but the compound is understood to also include its zwitterionic form. Embodiments herein may also exclude one or more of the compounds. Compounds may be identified either by their chemical structure or their chemical name. In a case where the chemical structure and chemical name would conflict, the chemical structure will prevail.

Unless otherwise stated, structures depicted herein are also meant to include all isomeric (e.g., enantiomeric, diastereomeric, and geometric (or conformational)) forms of the structure when applicable; for example, the R and S configurations for each asymmetric center. Therefore, single stereochemical isomers as well as enantiomeric, diastereomeric, and geometric (or conformational) mixtures of the present compounds are within the scope of the present description. The therapeutic compounds unless otherwise noted, also encompasses all possible tautomeric forms of the illustrated compound, if any. The term also includes isotopically labeled compounds where one or more atoms have an atomic mass different from the atomic mass most abundantly found in nature. Examples of isotopes that may be incorporated into the present compounds include, but are not limited to, ²H (D), ³H (T), ¹¹C, ¹³C, ¹⁴C, ¹⁵N, ¹⁸O, ¹⁷O, any one of the isotopes of sulfur, etc. The compounds may also exist in unsolvated forms as well as solvated forms, including hydrated forms. The compounds may exist in multiple crystalline or amorphous forms. However, amorphous or substantially amorphous forms are preferred for the formulations contemplated herein.

The expression "amorphous" generally designates a non-crystalline state lacking the long-range order characteristic of a crystal. The expression "substantially amorphous" as used herein refers to a solid state which is mainly in an amorphous state, for instance, containing less than 5% by weight of crystalline solid. The amorphous nature of a solid may be determined by standard methods, including X-ray powder diffraction (XRPD), Differential Scanning Calorimetry (DSC) or Fourier transform (FT) Raman spectroscopy.

Where a particular enantiomer is preferred, it may, in some embodiments be provided substantially free of the corresponding enantiomer and may also be enantiomerically enriched.

"Enantiomerically enriched" means that the compound is made up of a significantly greater proportion of one enantiomer. In certain embodiments the compound is made up of at least about 90% by weight of a preferred enantiomer. In other embodiments the compound is made up of at least about 95%, 98%, or 99% by weight of a preferred enantiomer. Preferred enantiomers may be isolated from racemic mixtures by any method known to those skilled in the art, including highpressure liquid chromatography (HPLC) or supercritical Fluid Chromatography (SFC) on chiral support, or by the formation and crystallization of chiral salts or be prepared by asymmetric syntheses.

The expression "pharmaceutically acceptable salt" refers to those salts of the compounds of the present description which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, S. M. Berge, et al. describes pharmaceutically acceptable salts in detail in J. Pharmaceutical Sciences, 66: 1-19 (1977). The salts can be prepared *in situ* during the final isolation and purification of the compounds of the present description, or separately by reacting a free base function of the compound with a suitable organic or inorganic acid (acid addition salts) or by reacting an acidic function of the compound with a suitable organic or inorganic base (base-addition salts).

The term "solvate" refers to a physical association of one of the present compound with one or more solvent molecules, including water and non-aqueous solvent molecules. This physical association may include hydrogen bonding. In certain instances, the solvate will be capable of isolation, for example when one or more solvent molecules are incorporated in the crystal lattice of a crystalline solid. The term "solvate" encompasses both solution-phase and isolable solvates. Exemplary solvates include, without limitation, hydrates, hemihydrates, ethanolates, hemiethanolates, n-propanolates, iso-propanolates, 1-butanolates, 2-butanolate, and solvates of other physiologically acceptable solvents, such as the Class 3 solvents described in the International Conference on Harmonization (ICH), Guide for Industry, Q3C Impurities: Residual Solvents (1997). Accordingly, the compound as herein described also includes each of its solvates and mixtures thereof.

As used herein, the expression "pharmaceutically acceptable ester" refers to esters of the compounds formed by the process of the present description which may hydrolyze *in vivo* and include those that break down readily in the human body to leave the parent compound or a salt thereof. Suitable ester groups include, for example, those derived from pharmaceutically acceptable aliphatic carboxylic acids, particularly alkanoic, alkenoic, cycloalkanoic and alkanedioic acids, in which each alkyl or alkenyl moiety advantageously has not more than 6 carbon atoms. Examples of particular esters include, but are not limited to, formates, acetates, propionates, butyrates, acrylates and ethylsuccinates of hydroxyl groups, and alkyl esters of an acidic group. Other ester groups include sulfonate or sulfate esters.

The expression "pharmaceutically acceptable prodrugs" as used herein refers to those prodrugs of the compounds formed by the process of the present description which are, within the scope of sound medical judgment, suitable for use in contact with the tissues of humans and lower animals with undue toxicity, irritation, allergic response, and the like, commensurate with a reasonable benefit/risk ratio, and effective for their intended use. "Prodrug", as used herein means a compound which is convertible in vivo by metabolic means (e.g. by hydrolysis) to afford any compound delineated by the formulae of the instant description.

Abbreviations may also be used throughout the application, unless otherwise noted, such abbreviations are intended to have the meaning generally understood by the field. Examples of such abbreviations include Me (methyl), Et (ethyl), Pr (propyl), i-Pr (isopropyl), Bu (butyl), t-Bu (tert-butyl), i-Bu (iso-butyl), s-Bu (sec-butyl), c-Bu (cyclobutyl), Ph (phenyl), Bn (benzyl), Bz (benzoyl), CBz or Cbz or Z (carbobenzyloxy), Boc or BOC (tert-butoxycarbonyl), and Su or Suc (succinimide).

The number of carbon atoms in a hydrocarbon substituent can be indicated by the prefix "Cₓ-C_{y}" or "Cₓ-_{y}" where x is the minimum and y is the maximum number of carbon atoms in the substituent. However, when the prefix "Cₓ-C_{y}" or "Cₓ-_{y}" is associated with a group incorporating one or more heteroatom(s) by definition (e.g. heterocycloalkyl, heteroaryl, etc), then x and y define respectively the minimum and maximum number of atoms in the cycle, including carbon atoms as well as heteroatom(s).

The term "alkyl" as used herein, refers to a saturated, straight- or branched-chain hydrocarbon radical typically containing from 1 to 20 carbon atoms. For example, "C₁-C₈ alkyl" contains from one to eight carbon atoms. Examples of alkyl radicals include, but are not limited to, methyl, ethyl, propyl, isopropyl, n-butyl, *tert*-butyl, neopentyl, n-hexyl, heptyl, octyl radicals and the like.

The term "alkenyl" as used herein, denotes a straight- or branched-chain hydrocarbon radical containing one or more double bonds and typically from 2 to 20 carbon atoms. For example, "C₂-₈ alkenyl" contains from two to eight carbon atoms. Alkenyl groups include, but are not limited to, for example, ethenyl, propenyl, butenyl, I-methyl-2-buten-I-yl, heptenyl, octenyl and the like.

The term "alkynyl" as used herein, denotes a straight- or branched-chain hydrocarbon radical containing one or more triple bonds and typically from 2 to 20 carbon atoms. For example, "C₂-₈ alkynyl" contains from two to eight carbon atoms. Representative alkynyl groups include, but are not limited to, for example, ethynyl,1-propynyl, 1-butynyl, heptynyl, octynyl and the like.

The terms "cycloalkyl", "alicyclic", "carbocyclic" and equivalent expressions refer to a group comprising a saturated or partially unsaturated (non-aromatic) carbocyclic ring in a monocyclic or polycyclic ring system, including spiro (sharing one atom), fused (sharing at least one bond) or bridged (sharing two or more bonds) carbocyclic ring systems, having from three to fifteen ring members. Examples of cycloalkyl groups include, without limitation, cyclopropyl, cyclobutyl, cyclopentyl, cyclopenten-1-yl, cyclopenten-2-yl, cyclopenten-3-yl, cyclohexyl, cyclohexen-1-yl, cyclohexen-2-yl, cyclohexen-3-yl, cycloheptyl, bicyclo[4,3,0]nonanyl, norbornyl, and the like. The term cycloalkyl includes both unsubstituted cycloalkyl groups and substituted cycloalkyl groups. The term "C₃-Cₙcycloalkyl" refers to a cycloalkyl group having from 3 to the indicated "n" number of carbon atoms in the ring structure. Unless the number of carbons is otherwise specified, "lower cycloalkyl" groups as herein used, have at least 3 and equal or less than 8 carbon atoms in their ring structure.

As used herein, the terms "heterocycle", "heterocycloalkyl", "heterocyclyl", "heterocyclic radical", and "heterocyclic ring" are used interchangeably and refer to a chemically stable 3- to 7-membered monocyclic or 7-10-membered bicyclic heterocyclic moiety that is either saturated or partially unsaturated, and having, in addition to carbon atoms, one or more, preferably one to four, heteroatoms, as defined above. When used in reference to a ring atom of a heterocycle, the term "nitrogen" includes a substituted nitrogen. As an example, in a saturated or partially unsaturated ring having 1-3 heteroatoms selected from oxygen, sulfur or nitrogen, the nitrogen may be N (as in 3,4-dihydro-2H-pyrrolyl), NH (as in pyrrolidinyl), or NR (as in N-substituted pyrrolidinyl). A heterocyclic ring can be attached to its pendant group at any heteroatom or carbon atom that results in a chemically stable structure and any of the ring atoms can be optionally substituted. Examples of heterocycloalkyl groups include, but are not limited to, 1,3-dioxolanyl, pyrrolidinyl, pyrrolidonyl, pyrazolinyl, pyrazolidinyl, imidazolinyl, imidazolidinyl, piperidinyl, piperazinyl, oxazolidinyl, isoxazolidinyl, morpholinyl, thiazolidinyl, isothiazolidinyl, tetrahydrofuranyl, tetrahydropyranyl, tetrahydrothiopyranyl, tetrahydrodithienyl, tetrahydrothienyl, thiomorpholino, thioxanyl, azetidinyl, oxetanyl, thietanyl, homopiperidinyl, oxepanyl, thiepanyl, oxazepinyl, diazepinyl, thiazepinyl, 1,2,3,6-tetrahydropyridinyl, 2-pyrrolinyl, 3-pyrrolinyl, 2H-pyranyl, 4H-pyranyl, dioxanyl, dithianyl, dithiolanyl, dihydropyranyl, dihydrothienyl, dihydrofuranyl, 3-azabicyclo[3,1,0]hexanyl, 3-azabicyclo[4,1,0]heptanyl, quinolizinyl, quinuclidinyl, tetrahydroquinolinyl, tetrahydroisoquinolinyl, decahydroquinolinyl, and the like. Heterocyclic groups also include groups in which a heterocyclic ring is fused to one or more aryl, heteroaryl, or cycloaliphatic rings, such as indolinyl, 3H-indolyl, chromanyl, chromenyl, phenanthridinyl, 2-azabicyclo[2.2.1]heptanyl, octahydroindolyl, or tetrahydroquinolinyl, where the radical or point of attachment is on the heterocyclyl ring. A heterocyclyl group may be mono- or bicyclic. The term "heterocyclylalkyl" refers to an alkyl group substituted by a heterocyclyl, wherein the alkyl and heterocyclyl portions independently are optionally substituted. The term "C₃-ₙheterocycloalkyl" refers to a heterocycloalkyl group having from 3 to the indicated "n" number of atoms in the ring structure, including carbon atoms and heteroatoms.

As used herein, the term "partially unsaturated" refers to a ring moiety that includes at least one double or triple bond between ring atoms but is not aromatic. The term "partially unsaturated" is intended to encompass rings having multiple sites of unsaturation but is not intended to include aryl or heteroaryl moieties, as herein defined.

The term "aryl" used alone or as part of a larger moiety as in "aralkyl", "aralkoxy", "aryloxy", or "aryloxyalkyl", refers to aromatic groups having 4n+2 conjugated π(pi) electrons, wherein n is an integer from 1 to 3, in a monocyclic moiety or a bicyclic or tricyclic fused ring system having a total of six to 15 ring members, wherein at least one ring in the system is aromatic and wherein each ring in the system contains three to seven ring members. The term "aryl" may be used interchangeably with the term "aryl ring". In certain embodiments of the present description, "aryl" refers to an aromatic ring system which includes, but not limited to, phenyl, biphenyl, naphthyl, azulenyl, anthracyl and the like, which may bear one or more substituents. The term "aralkyl" or "arylalkyl" refers to an alkyl residue attached to an aryl ring. Examples of aralkyl include, but are not limited to, benzyl, phenethyl, and the like. Also included within the scope of the term "aryl", as it is used herein, is a group in which an aromatic ring is fused to one or more non-aromatic rings, such as indanyl, indenyl, phthalimidyl, naphthimidyl, fluorenyl, phenanthridinyl, or tetrahydronaphthyl, and the like. The term "C₆-ₙaryl" refers to a aryl group having from 6 to the indicated "n" number of atoms in the ring structure.

The term "heteroaryl", used alone or as part of a larger moiety, e.g., "heteroaralkyl", or "heteroaralkoxy", refers to aromatic groups having 4n+2 conjugated π(pi) electrons, wherein n is an integer from 1 to 3 (e.g. having 5 to 18 ring atoms, preferably 5, 6, or 9 ring atoms; having 6, 10, or 14 π electrons shared in a cyclic array); and having, in addition to carbon atoms, from one to five heteroatoms. The term "heteroatom" includes but is not limited to nitrogen, oxygen, or sulfur, and includes any oxidized form of nitrogen or sulfur, and any quaternized form of a basic nitrogen. A heteroaryl may be a single ring, or two or more fused rings. The term "heteroaryl", as used herein, also includes groups in which a heteroaromatic ring is fused to one or more aryl, cycloaliphatic, or heterocyclic rings, where the radical or point of attachment is on the heteroaromatic ring. Nonlimiting examples of heteroaryl groups include thienyl, furanyl (furyl), pyrrolyl, imidazolyl, pyrazolyl, triazolyl, tetrazolyl, oxazolyl, isoxazolyl, oxadiazolyl, thiazolyl, isothiazolyl, thiadiazolyl, pyridyl, pyridazinyl, pyrimidinyl, pyrazinyl, triazinyl, indolyl, 3H-indolyl, isoindolyl, indolizinyl, benzothienyl (benzothiophenyl), benzofuranyl, dibenzofuranyl, indazolyl, benzimidazolyl, benzoxazolyl, benzothiazolyl, benzotriazolyl, pyrrolopyridinyl (e.g. pyrrolo[3,2-b]pyridinyl or pyrrolo[3,2-c]pyridinyl), pyrazolopyridinyl (e.g. pyrazolo[1,5-a]pyridinyl), furopyridinyl, purinyl, imidazopyrazinyl (e.g. imidazo[4,5-b]pyrazinyl), quinolyl (quinolinyl), isoquinolyl (isoquinolinyl), quinolonyl, isoquinolonyl, cinnolinyl, phthalazinyl, quinazolinyl, quinoxalinyl, 4H-quinolizinyl, naphthyridinyl, and pteridinyl carbazolyl, acridinyl, phenanthridinyl, phenazinyl, phenothiazinyl, phenoxazinyl, tetrahydroquinolinyl, tetrahydroisoquinolinyl, and pyrido[2,3-b]-1,4-oxazin-3(4H)-one. A heteroaryl group may be mono- or bicyclic. Heteroaryl groups include rings that are optionally substituted. The term "heteroaralkyl" refers to an alkyl group substituted by a heteroaryl, wherein the alkyl and heteroaryl portions independently are optionally substituted. Examples include, but are not limited to, pyridinylmethyl, pyrimidinylethyl and the like. For instance, the term "C₅-ₙheteroaryl" refers to a heteroaryl group having from 5 to the indicated "n" number of atoms in the ring structure, including carbon atoms and heteroatoms. The term "halogen" designates a halogen atom, i.e. a fluorine, chlorine, bromine or iodine atom, preferably fluorine or chlorine.

During development efforts, it was discovered that the compounds herein, especially Compound 1 below, are essentially insoluble when in crystalline form, resulting in a very poor oral bioavailability. Efforts have thus been directed to the identification of a suitable process and formulation to achieve substantially enhanced oral bioavailability.

The present technology therefore relates to the preparation of a solid dispersion of an amorphous compound in a solid matrix which prevents the compound from reverting to its crystalline form, to these solid dispersions, to compositions and solid dosage forms comprising them as well as their medical uses. The solid dispersion will be, for instance, in the form of particles, powder, etc.

The compounds contemplated for the present solid dispersions are as defined in U.S. Patent No. 9,765,031, and including those defined herein in the following paragraphs. When referring to chemical moieties, the recitation of a listing of chemical groups in any definition of a variable includes definitions of that variable as any single group or combination of listed groups. Similarly, the recitation of an embodiment herein includes that embodiment as any single embodiment or in combination with any other embodiments or portions thereof. As such, the following embodiments are present alone or in combination if applicable.

Examples of compound are described in U.S. Patent No. 9,765,031.

Compounds other than Compound 1 and Compound 7 are reference compounds. For instance, the compounds are selected from Compounds 1 to 26, or a tautomer or salt thereof:

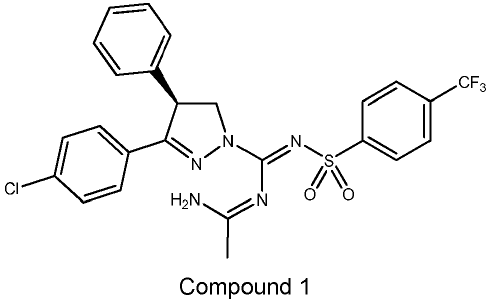

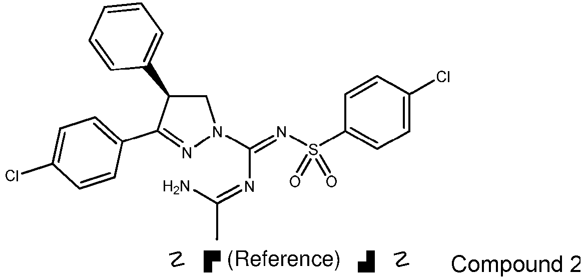

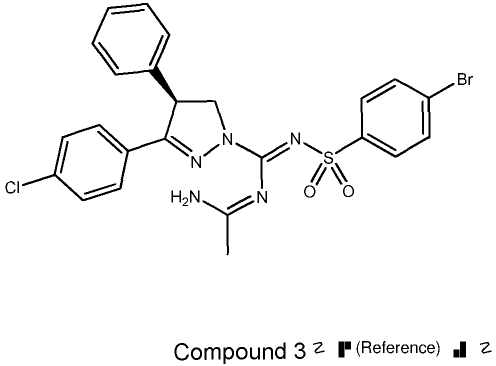

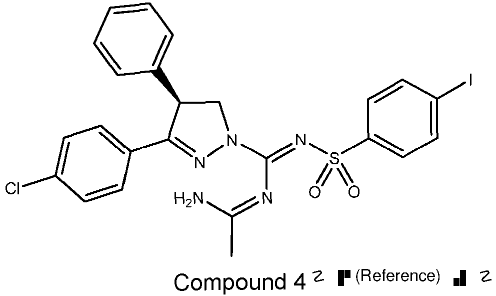

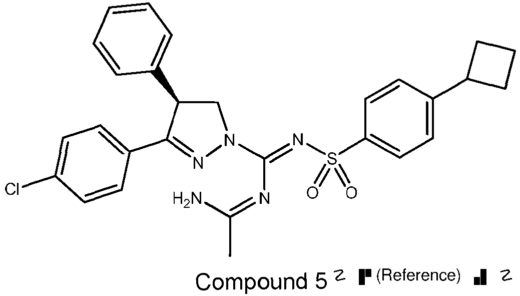

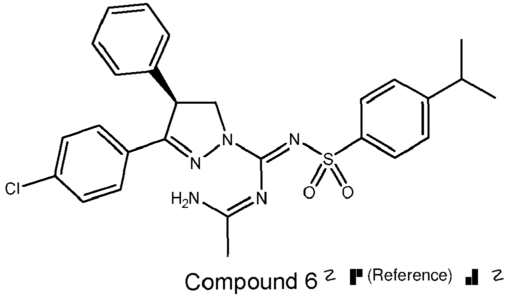

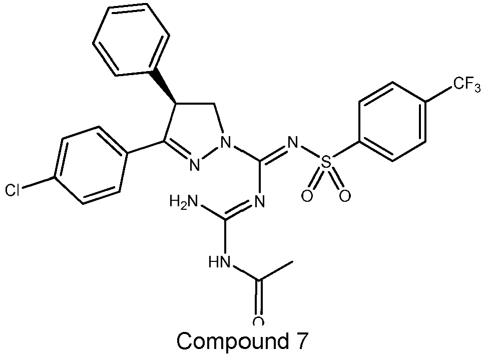

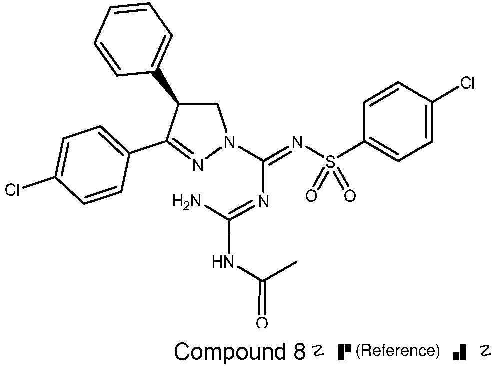

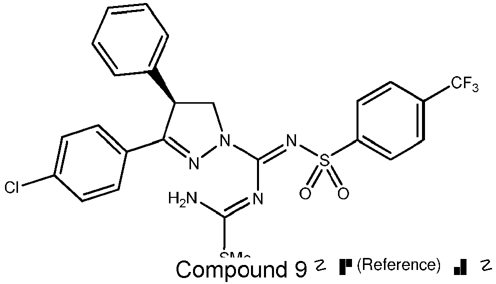

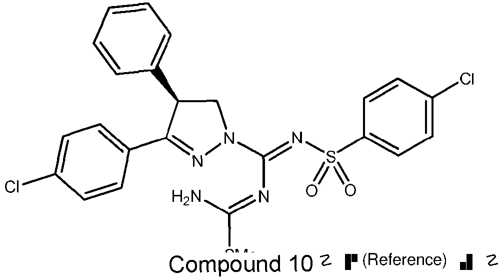

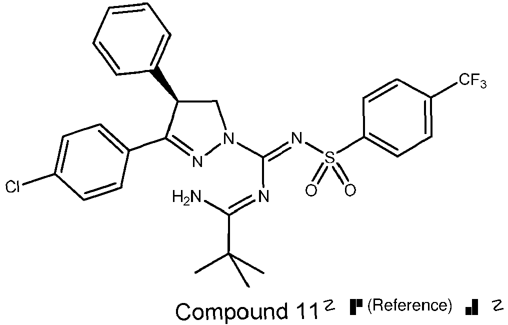

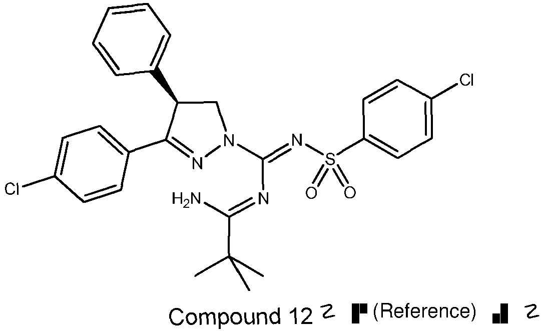

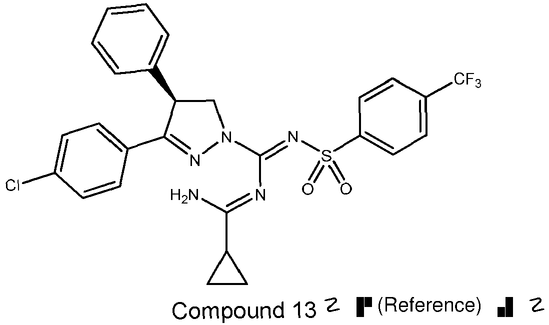

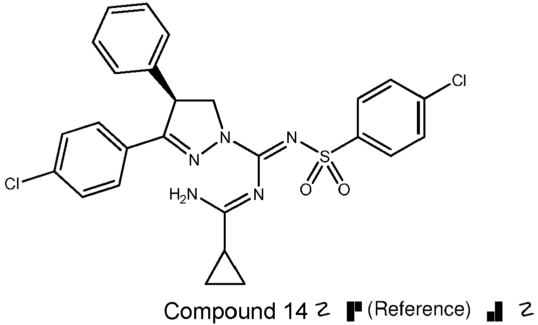

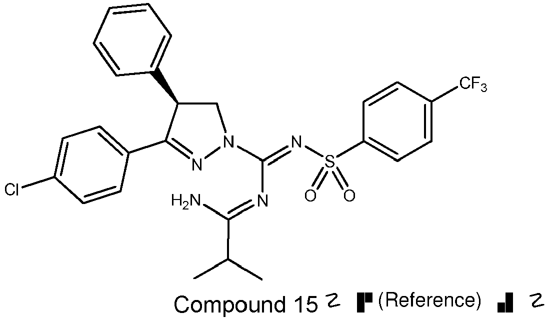

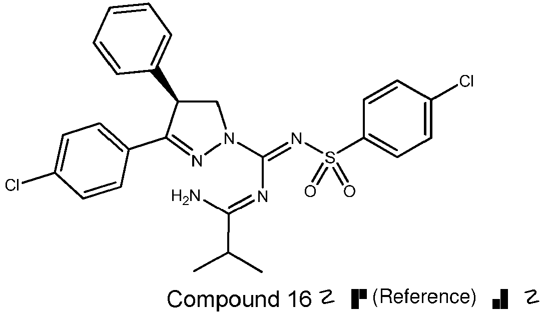

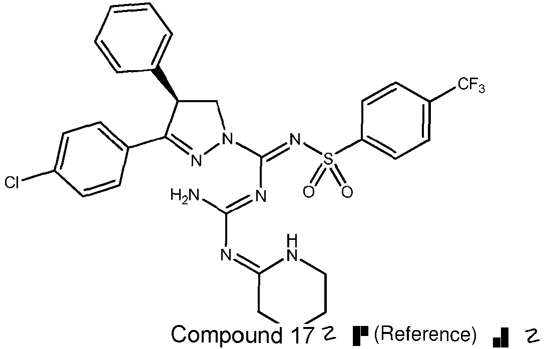

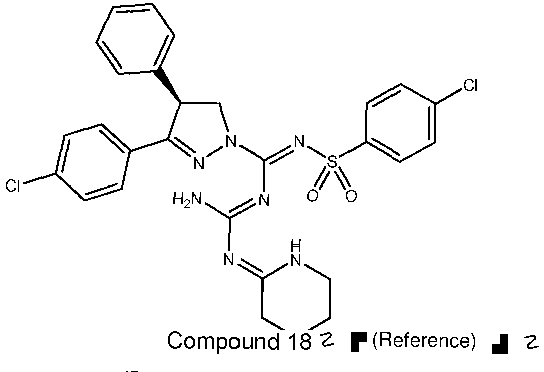

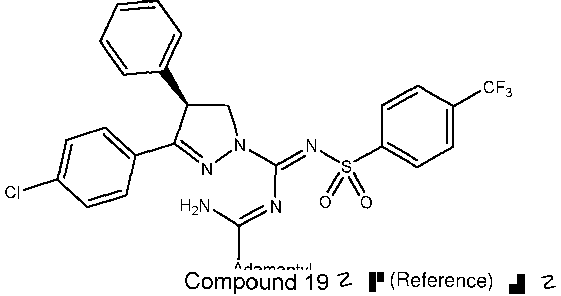

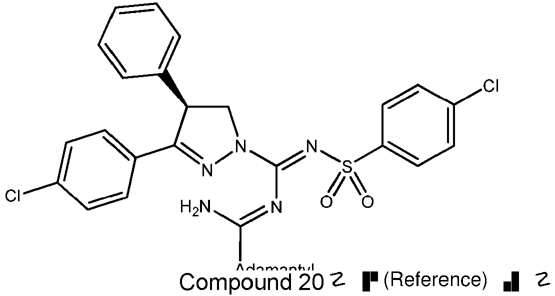

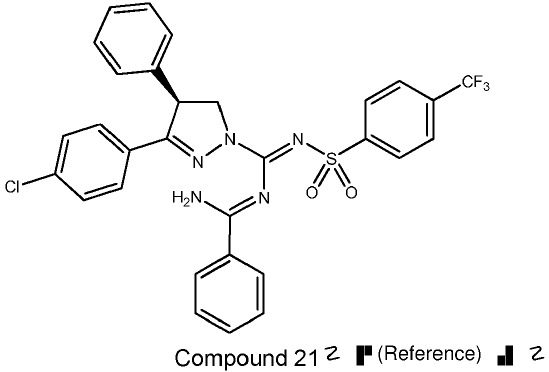

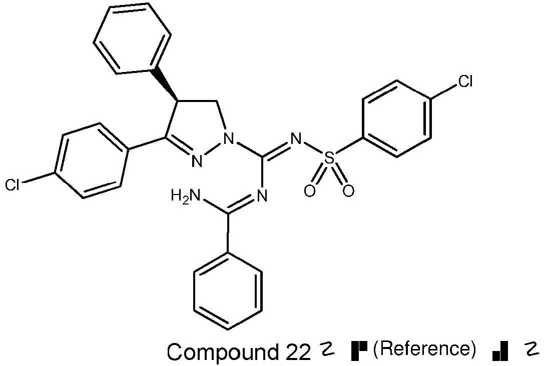

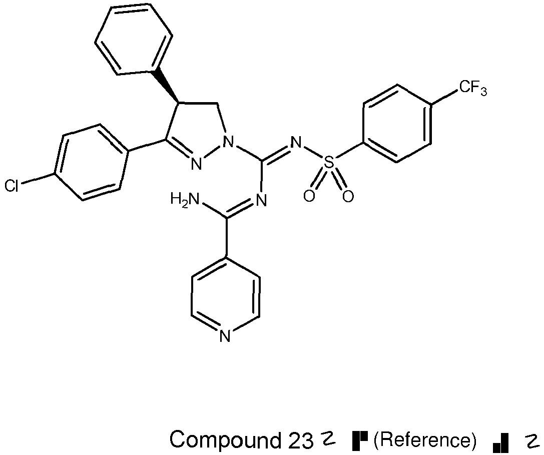

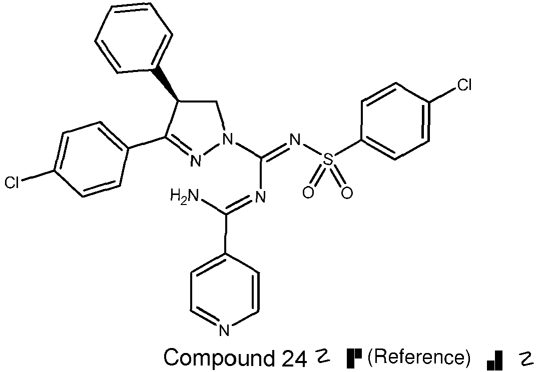

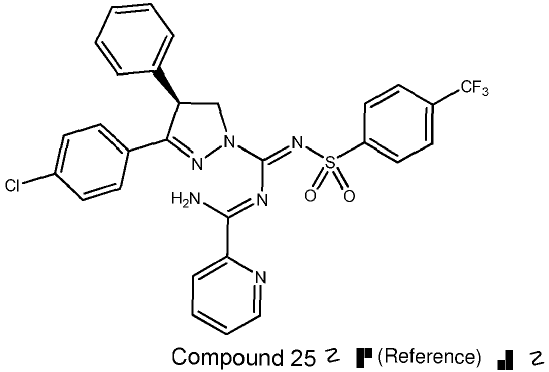

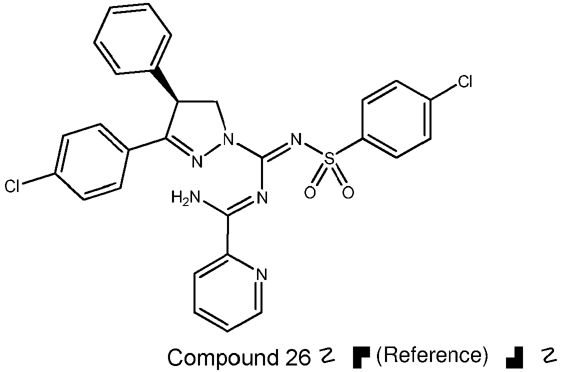

These compounds may be prepared by conventional chemical synthesis such as those described in U.S. Patent No. 9,765,031. As can be appreciated by the skilled artisan, further methods of synthesizing the compounds of the formulae herein will be evident to those of ordinary skill in the art. Additionally, the various synthetic steps may be performed in an alternate sequence or order to give the desired compounds.

As indicated above, the compound will be in a substantially amorphous state when included in the present solid dispersion. For example, the compound in the solid dispersion comprises less than 5% of crystalline form, or less than 2% of crystalline form, or less than 1% of crystalline form, or even less than 0.5% of crystalline form.

The compound is dispersed in a matrix which prevents its reconversion to a crystalline state. Preferably, the matrix comprises a pharmaceutically acceptable polymer having a glass transition temperature of at least 50°C, or at least 80°C, or at least 100°C, or at least 120°C, or at least 140°C, or between 50°C and 200°C, or between 80°C and 200°C, or between 100°C and 180°C. Examples of polymers include polyvinylpyrrolidones and copolymers thereof, cellulose and cellulose derivatives, polyethylene glycol, polylactic acid and polymethacrylate having a glass transition temperature of at least 50°C. These polymers should also be soluble in an organic solvent and be chemically stable over time. For instance, the polymer is a polyvinylpyrrolidone or a copolymer thereof (e.g. a N-vinyl-2-pyrrolidone and vinyl acetate copolymer) having an average molecular weight of between 5,000 and 100,000. Alternatively, the polymer is a cellulose derivative such as an esterified hydroxyalkyl methylcellulose, of which hydroxypropyl methylcellulose acetate succinate is an example.

The weight ratio of polymer and compound in the solid dispersion may be adjusted to achieve a stable amorphous state of the compound and will vary depending on the compound. Examples of polymer:compound weight ratios are between between 1:1 to 6:1, or between 1:1 and 4:1, or between 2:1 and 5:1, or between 3:1 and 5:1, or between 1:1 and 3:1.

In some preferred alternatives, the solid dispersion further comprises a pharmaceutically acceptable surfactant. Examples of surfactant includes long chain alkyl or alkenyl sulfate salts (e.g. sulfates of C₈ to C₂₀ alcohols, such as sodium lauryl sulfate, sodium laureth sulfate, ammonium lauryl sulfate, sodium pareth sulfate, and the like); alkyl sulfonate salts (e.g. perfluorooctanesulfonate, perfluorobutanesulfonate, docusate, and the like); sorbitan long chain carboxylic acid esters (e.g. C₈ to C₂₀ carboxylic acids such as oleate, stearate, laurate, and the like); pegylated sorbitan long chain carboxylic acid esters (e.g. Tween 20, Tween 40, Tween 60, Tween 80); polyethylene-polypropylene glycol block copolymers (e.g. poloxamers); pegylated or non-pegylated mono, di, and tri glyceride long chain carboxylic acid esters (e.g. PEG-4, -6 or -8, C₈ to C₂₀ alkyl carboxylic acid triglycerides); polyethylene and/or polypropylene glycol alkoxylates; alkylphenol alkoxylates; alkylphenol derivatives of polyethylene and/or polypropylene glycols (e.g. Triton X-100); and sucrose long chain carboxylic acid esters, or combinations thereof. For instance, the surfactant is a long chain alkyl sulfate, such as sodium lauryl sulfate, sodium laureth sulfate, ammonium lauryl sulfate, sodium pareth sulfate, and the like.

When the surfactant is present, the weight ratio of polymer to surfactant in the solid dispersion will be adjusted to obtain a homogeneous dispersion, and will depend on the compound, polymer and surfactant used. Examples of polymer:surfactant weight ratios range from 5:1 to 20:1, or of 10:1 to 15:1.

The present solid dispersions may be prepared by dry or wet mixing methods. For instance, methods include such as rapid acoustic mixing, extrusion, planetary mixing, ball milling, and other similar mixing methods, or may be dissolved together with the polymer and the optional surfactant and dried by a method such as spray drying to form the solid dispersion.

The solid dispersion as defined herein can be formulated in a solid oral pharmaceutical composition for administration to a subject, the solid dispersion being optionally admixed with a pharmaceutically acceptable carrier, diluent, or excipient. In some examples, the active compound is present in the oral formulation at a concentration of between 5 wt.% and 50 wt.%, or between 10 wt.% and 40 wt.%.

The expression "pharmaceutically acceptable carrier, diluent, or excipient" and equivalent expressions, refer to a non-toxic carrier, diluent, or excipient that does not destroy the pharmacological activity of the compound or the integrity of the solid dispersion with which it is formulated. Pharmaceutically acceptable carriers, diluents or excipients that may be used in the compositions of this disclosure include, but are not limited to, binders, sweeteners, disintegrating agents, diluents, flavorings, coating agents, preservatives, lubricants, and/or polymer.

Examples of binders include cellulose-based substances such as microcrystalline cellulose and carboxymethylcellulose, and other binders like gum acacia, gelatin, corn starch, gum tragacanth, sodium alginate, or polyethylene glycol (PEG). Examples of sweeteners include sucrose, lactose, glucose, aspartame or saccharine. Disintegrating agents include corn starch, methylcellulose, polyvinylpyrrolidone, xanthan gum, bentonite, alginic acid or agar. Examples of diluents include lactose, sorbitol, mannitol, dextrose, kaolin, cellulose, calcium carbonate, calcium silicate or dicalcium phosphate. Flavoring agents include peppermint oil, oil of wintergreen, cherry, orange, or raspberry flavoring. Coating agents include polymers or copolymers of acrylic acid and/or methacrylic acid and/or their esters, waxes, fatty alcohols, zein, shellac or gluten. Suitable preservatives include sodium benzoate, vitamin E, alpha-tocopherol, ascorbic acid, methyl paraben, propyl paraben or sodium bisulphite. Suitable lubricants include magnesium stearate, stearic acid, sodium oleate, sodium chloride or talc. Examples of excipients may further include a polymer selected from the group consisting of polyvinylpyrrolidone (PVP), polyvinylpyrrolidone-vinylacetate copolymer (PVP-VA), hydroxypropylmethylcellulose (HPMC), hypromellose-acetate-succinate (HPMCAS), and mixtures thereof.

For instance, solid dosage forms for oral administration include capsules, tablets, pills, and granules. In a preferred alternative, the composition is a solid dosage form which comprises the solid dispersion as described herein and at least one binder as defined in the preceding paragraph, the binder preferably comprising microcrystalline cellulose.

The solid dispersions and compositions may also be employed as fillers in soft and hard-filled capsules. The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that release the active ingredient(s) only, or preferentially, in a certain part of the intestinal tract, optionally, in a delayed manner. Examples of embedding compositions that can be used include polymeric substances and waxes. The composition may also be in microencapsulated form with one or more excipients as noted above.

As used herein, the term "effective amount" means that amount of a drug or pharmaceutical agent that will elicit the biological or medical response of a tissue, system, animal or human that is being sought, for instance, by a researcher or clinician. Furthermore, the term "therapeutically effective amount" means any amount which, as compared to a corresponding subject who has not received such amount, results in treatment, healing, prevention, or amelioration of a disease, disorder, or symptom thereof, or a decrease in the rate of advancement of a disease or disorder. The term also includes within its scope amounts effective to enhance normal physiological function.

As used herein, the terms "treatment," "treat," and "treating" refer to reversing, alleviating, delaying the onset of, or inhibiting the progress of a disease or disorder, or one or more symptoms thereof, as described herein. In some embodiments, treatment may be administered after one or more symptoms have developed. In other embodiments, treatment may be administered in the absence of symptoms. For example, treatment may be administered to a susceptible individual prior to the onset of symptoms (e.g., in light of a history of symptoms and/or in light of genetic or other susceptibility factors). Treatment may also be continued after symptoms have resolved, for example to prevent or delay their recurrence.

The term "patient" or "subject" as used herein refers to an animal such as a mammal. A subject may therefore refer to, for example, mice, rats, dogs, cats, horses, cows, pigs, guinea pigs, primates including humans and the like. Preferably the subject is a human.

The compounds included in the present solid dispersions are useful for the treatment of diseases and disorders where inhibition of the cannabinoid receptor CB₁ is indicated, for instance such as those described in U.S. Patent No. 9,765,031. Such diseases and disorders are generally related to diabetes and metabolic disorders (e.g. metabolic syndrome). Preferably, the active ingredient selective targets the CB₁ receptor in peripheral tissue (e.g. adipose tissue, liver, muscle, lung, kidney, macrophages, pancreatic beta cells and gastrointestinal tract), while not interacting with CB₁ receptors in brain tissue, thereby avoiding or reducing CNS-related side effects.

The effect of the present compounds may include reduced food intake, reduced body weight, reversed insulin and leptin resistance, reverse hepatic steatosis (fatty liver) and improved dyslipidemia. Examples of diseases and disorders to be treated include obesity, diabetes (type I or II), non-alcoholic and alcoholic fatty liver disease (a risk factor for insulin resistance), a co-morbidity of obesity, a co-morbidity of diabetes, Prader-Willi Syndrome (PWS), Pro-opiomelanocortin (POMC) deficiency obesity, LepR deficiency obesity, POMC heterozygous deficiency obesity, POMC epigenetic disorders, Bardet-Biedl syndrome, Alström syndrome, dyslipidemia predisposing to arteriosclerotic heart disease, diabetic nephropathy, fibrosis and fibrotic diseases such as Idiopathic Pulmonary Fibrosis (IPF) and Hermansky-Pudlak Syndrome pulmonary fibrosis (HPS-PF), and gout. For instance, the co-morbidity of obesity is selected from metabolic syndrome, dementia, heart disease, hypertension, gallbladder disease, gastrointestinal disorders, menstrual irregularities, degenerative arthritis, venous statis ulcer, pulmonary hypoventilation syndrome, sleep apnea, snoring, coronary artery disease, arterial sclerotic disease, pseudotumor cerebri, osteoarthritis, high cholesterol, and increased incidence of malignancies of the liver, ovaries, cervix, uterus, breasts, prostate, or gallbladder. In preferred examples, the disease or disorder include diabetes (type 1 or 2), obesity, and non-alcoholic fatty liver disease (e.g. non-alcoholic steatohepatitis). Examples of co-morbidities of diabetes (e.g. type l) include diabetic nephropathy, chronic kidney disease, diabetic retinopathy, and peripheral and autonomic neuropathy.

The present solid dispersions and compositions may also be used in a method for preventing or reversing the deposition of adipose tissue in a subject, which is expected to contribute to a reduction of incidence or severity of obesity, which in turn would reduce the incidence or severity of associated co-morbidities.

The present description provides a method of treating a disorder (as described herein) in a subject, comprising administering to the subject identified as in need thereof, a solid dispersion or composition of the present description. Any references to methods of treatment by therapy or surgery or *in vivo* diagnosis methods of this description is to be interpreted as a reference to compounds, pharmaceutical compositions and medicaments of the present invention for use in those methods.

The identification of those patients who are in need of treatment for the disorders described above is well within the ability and knowledge of one skilled in the art. Certain of the methods for identification of patients which are at risk of developing the above disorders which can be treated by the subject method are appreciated in the medical arts, such as family history, and the presence of risk factors associated with the development of that disease state in the subject patient. A clinician skilled in the art can readily identify such candidate patients, by the use of, for example, clinical tests, physical examination, medical/family history, and genetic determination.

A method of assessing the efficacy of a treatment in a subject includes determining the pretreatment symptoms of a disorder by methods well known in the art and then administering a therapeutically effective amount of a compound of the present description, to the subject. After an appropriate period of time following the administration of the compound (e.g., 1 week, 2 weeks, one month, six months), the symptoms of the disorder are determined again. The modulation (e.g., decrease) of symptoms and/or of a biomarker of the disorder indicates efficacy of the treatment. The symptoms and/or biomarker of the disorder may be determined periodically throughout treatment. For example, the symptoms and/or biomarker of the disorder may be checked every few days, weeks or months to assess the further efficacy of the treatment. A decrease in symptoms and/or biomarker of the disorder indicates that the treatment is efficacious.

Pharmaceutical compositions provided herein are adapted for oral administration. Such formulations may be administered with or without food. The solid dispersions or compositions are formulated in unit dosage forma for ease of administration and uniformity of dosage. The expression "unit dosage form" as used herein refers to a physically discrete unit of agent appropriate for the patient to be treated. It will be understood, however, that the total daily usage of the solid dispersions and compositions of the present disclosure will be decided by the attending physician within the scope of sound medical judgment.

The amount of solid dispersion that may be included in a single dosage form will vary depending upon the patient to be treated (e.g. child vs adult, etc.) and the particular compound included in the dispersion. Provided compositions may be formulated such that a total daily dosage of, for instance, between 0.01 and 20 mg/kg body weight/day of the compound can be administered to a patient receiving these compositions. Single dose compositions may contain such an amount, or the total daily dose may be divided in multiple dosage forms to be taken, for instance, one, two or three times a day. For instance, a single dose may include between 5 and 500 mg of the active ingredient, or between 20 and 200 mg. Treatment regimens may comprise administration to a patient a total amount of from about 10 mg to about 1000 mg of the compound(s) of the present description per day in a single dose or divided in multiple doses.

It will be understood, that the total daily dose of the compound will be decided by the attending physician within the scope of sound medical judgment. For instance, a specific dosage or treatment regimen for any particular patient will depend upon a variety of factors, including age, body weight, general health, sex, diet, time of administration, rate of excretion, drug combination, the judgment of the treating physician, and the severity of the symptoms associated with the disease or disorder.

Depending upon the disease or disorder to be treated, additional therapeutic agents may also be present in the compositions of this disclosure or co-administered separately. Non-limiting examples of additional therapeutic agents which could be used in combination with the present solid dispersions and formulations include antidiabetic agents, cholesterol-lowering agents, antiinflammatory agents, antimicrobial agents, matrix metalloproteinase inhibitors, lipoxygenase inhibitors, cytokine antagonists, immunosuppressants, anti-cancer agents, anti-viral agents, cytokines, growth factors, immunomodulators, prostaglandins, or anti-vascular hyperproliferation compound. The treatment may also be complemented with other treatments or interventions such as surgery, radiotherapy (e.g., gamma-radiation, neutron beam radiotherapy, electron beam radiotherapy, proton therapy, brachytherapy, and systemic radioactive isotopes), a biologic response modifier (e.g., an interferon, an interleukin, tumor necrosis factor (TNF)), and agents used to attenuate an adverse effect of the present compound or of a co-administered ingredient.

The recitation of an embodiment for a variable herein includes that embodiment as any single embodiment or in combination with any other embodiments or portions thereof. The recitation of an embodiment herein includes that embodiment as any single embodiment or in combination with any other embodiments or portions thereof.

### EXAMPLES

The following non-limiting examples are illustrative embodiments and should not be construed as further limiting the scope of the present invention. These examples will be better understood with reference to the accompanying figures.

Unless otherwise indicated, all numbers expressing quantities of ingredients, reaction conditions, concentrations, properties, stabilities, and so forth used in the specification and claims are to be understood as being modified in all instances by the term "about." At the very least, each numerical parameter should at least be construed in light of the number of reported significant digits and by applying ordinary rounding techniques. Accordingly, unless indicated to the contrary, the numerical parameters set forth in the present specification and attached claims are approximations that may vary depending upon the properties sought to be obtained. Notwithstanding that the numerical ranges and parameters setting forth the broad scope of the embodiments are approximations, the numerical values set forth in the specific examples are reported as precisely as possible. Any numerical value, however, inherently contain certain errors resulting from variations in experiments, testing measurements, statistical analyses and such.

### Example 1 - Solid dispersions and compositions

### (a) Solid dispersion in molten polymer (F-MP for comparison)

Polysorbate 80 (Protameen Chemicals) was added to the molten PEG-8000 (Spectrum) and mixed for 2 minutes. Compound 1 was added and mixed vigorously using an impeller for 30 minutes at 60°C in a hot plate. The molten material was then spread across an aluminum foil. The solidified film of about 2-mm thickness was milled with a Quadro Comil (conical mil) equipped with a grated round 1575 µm opening screen followed by square screen 1143µm and round impeller, spacer 0.25 inch at 1200rpm. The F-MP formulation composition is detailed in Table 1.

**Table 1. Composition of F-MP**

| **Ingredient** | **% w/w** | **mg/capsule** |
|---|---|---|
| Compound 1 | 10 | 50 |
| PEG-8000 | 87.5 | 437.5 |
| Polysorbate 80 | 2.5 | 12.5 |
| **Total:** | **100** | **500** |

### (b) Solid self-emulsifying drug delivery system (F-SEDDS) (for comparison)

Kolliphor^{™} HS 15 (BASF) was dissolved in about 5 grams of water and mixed with Labrasol^{™} (Gattefossé). Using the high shear granulator GMX 0.1, Compound 1 was mixed with this solution at 60 rpm for 5 minutes. Magnesium aluminometasilicate (Neusilin^{™} UFL 2, by Fuji Chemical Industry Co., Ltd.) was added to the creamy mixture and mixed for 2 minutes at 325rpm/1800rpm impeller/chopper speeds. Low substituted hydroxypropyl cellulose (HPC, Nisso America, Inc.) was dissolved in remaining water and the solution was added at 10 g/min and mixed with the powder mixture at 325rpm/1800rpm impeller/chopper speeds. Due to the small batch size, the granules were dried in a conventional oven at 60°C for 2.5 hours. The dried material was granulated by hand using a using an 18-mesh sieve. The lubricant (sodium stearyl fumarate, JRS Pharma) was sieved through a 40-mesh sieve and mixed with granules using a V-blender at 25 rpm for 2 minutes. The F-SEDDS formulation composition is detailed in Table 2.

**Table 2. Composition of F-SEDDS**

| **Ingredient** | **% w/w** | **mg/capsule** |
|---|---|---|
| Compound 1 | 20 | 50 |
| Neusilin^{™} UFL 2 | 40 | 100 |
| Labrasol^{™} ALF | 26.7 | 66.7 |
| Kolliphor^{™} HS 15 | 5 | 12.5 |
| HPC | 5 | 12.5 |
| Water | (60) | - |
| Sodium stearyl fumarate | 3.3 | 8.3 |
| **Total:** | **100** | **250** |

| | | |
|---|---|---|
| (evaporated during the process) | | |

### (c) Solid dispersion in PVP (F1)

An amorphous co-precipitate was obtained from a solution composed of Compound 1 (1%), polyvinyl pyrrolidone (4%) (Plasdone^{™} K29/32, Ashland) and a 20:80 w/w mixture of dichloromethane/methanol (95%). The solution was spray dried using a Yamato Lab Spray Dryer Model GB22 with internal nozzle diameter 711 µm, inlet temperature 65°C, atomization air 0.1 MPa, drying air flow 0.5 m³/min and spray rate 20 g/min. The formulation was then prepared by mixing the amorphous co-precipitate with microcrystalline cellulose (Tabulose 102, Blanver) for 5 minutes at 25 rpm in a V-blender. Formulation F1's composition is summarized in Table 3.

**Table 3. Composition of F1**

| **Ingredient** | **% w/w** | **mg/capsule** |
|---|---|---|
| Compound 1/PVP | 12/55 | 50/228 |
| Microcrystalline cellulose | 33 | 139 |
| **Total:** | **100** | **417** |

### (d) Solid dispersion in PVP with surfactant (F2(a) to F2(e))

Formulations F2(a) to F2(e) were obtained by first dissolving 15 g of povidone (Plasdone K29/32) and 1 g of sodium lauryl sulfate (from Stepan) in 400 g of dichloromethane/methanol (20:80 v/v). Compound 1 (4 g) was added to the solution and dissolved. The solution was spray dried using a Yamato Lab Spray Dryer Model GB210 with internal nozzle diameter 711 µm, inlet temperature 65°C, atomization air 0.15 MPa, drying air flow 0.4 m³/min and spray rate 22 g/min.

The spray drying dispersion (SDD) was then spread on a tray and placed in a Fisher Scientific Istotemp Model 655F oven at 60°C for 60 minutes for a secondary drying step. The final blend was prepared by mixing for 5 minutes at 25 rpm in a V-blender 8.3 grams of SDD with 1.7 grams of microcrystalline cellulose (Tabulose 102). Cellulose was added in order to ensure proper disintegration of the capsule content due to strong binding properties of PVP. The final blend F2 was filled in HPMC "0" type or gelatin AAA type capsules to achieve various Compound 1 doses. The final F2(a) to F2(e) capsules compositions are shown in Table 4.

**Table 4. Composition of F2(a) to F2(e) capsules**

| **Ingredient** | **mg/capsule** | | | | |
|---|---|---|---|---|---|
| | **F2(a)** | **F2(b)** | **F2(c)** | **F2(d)*** | **F2(e)** |
| Compound 1 | 50 | 22 | 35 | 150 | 150 |
| Povidone | 187.5 | 82.5 | 131.25 | 562.5 | 562.5 |
| Sodium lauryl sulfate | 12.5 | 5.5 | 8.75 | 37.5 | 37.5 |
| Microcrystalline cellulose | 50 | 22 | 35 | 150 | 150 |
| Capsule type | HPMC "0" | HPMC "0" | HPMC "0" | HPMC "0" | Gelatin AAA |
| **Total:** | **300** | **132** | **210** | **900** | **900** |

| | | | | | |
|---|---|---|---|---|---|
| *Composition F2(d) includes 3 HPMC size "0" capsules, each containing 50 mg of Compound 1. | | | | | |

### (e) Solid dispersion in HPMC-AS (F3)

Hypromellose acetate succinate (16 g, HPMC-AS-MF, Ashland) was first dissolved in 400 g of dichloromethane/methanol (50:50 v/v). Compound 1 (4 g) was added to the solution and dissolved. The solution was spray dried using a Yamato Lab Spray Dryer Model GB210 with internal nozzle diameter 711 µm, inlet temperature 65°C, atomization air 0.15 MPa, drying air flow 0.4 m³/min and spray rate 22 g/min. No secondary drying was needed. The SDD was filled in HPMC capsules size 0. The final F3 capsule composition is shown in Table 5.

**Table 5. Composition of F3 capsules**

| **Ingredient** | **mg/capsule** |
|---|---|
| Compound 1 | 50 |
| HPMC-AS | 200 |
| **Total:** | **250** |

### (f) Solid dispersion in PVP with surfactant (F4)

The SDD used in this example is composed of same ingredients as the one prepared in (d) but with a higher drug load of 40%. Povidone and sodium lauryl sulfate were immediately dissolved in dichloromethane/methanol (20:80 v/v). To complete dissolution of Compound 1 was obtained by adding more dichloromethane (200 mL) to a final solvent system composition of 33:67 (v/v). The dissolved solids content was then of 3.3% (w/w). The solution was spray dried using a Yamato Lab Spray Dryer Model GB210 with internal nozzle diameter 711 µm, inlet temperature 65°C, atomization air 0.15 MPa, drying air flow 0.4 m³/min and spray rate 22 g/min. After spray drying the SDD was spread on a tray and placed in a Fisher Scientific Istotemp Model 655F oven at 60°C for 60 minutes for a secondary drying step. The final blend was prepared by mixing for 5 minutes at 25 rpm in a V-blender 8.3 grams of SDD with 1.7 grams of microcrystalline cellulose 102. The final blend was filled into HPMC capsules size 0 (Table 6).

**Table 6. Composition of F4 capsules**

| **Ingredient** | **mg/capsule** |
|---|---|
| Compound 1 | 50 |
| Povidone | 68.8 |
| Sodium lauryl sulfate | 6.3 |
| Microcrystalline cellulose | 24.9 |
| **Total:** | **150** |

### (g) Solid dispersion in Neusilin with surfactant (F5)

Compound 1 (4 g) was first dissolved in 400 grams of dichloromethane/methanol (50:50 v/v). Labrasol^{™} (8 g) was added to the solution and dissolved. Neusilin^{™} (8 g) was dispersed into the solution and mixed 15 minutes. The dispersion was spray dried using a Yamato Lab Spray Dryer Model GB210 with internal nozzle diameter 711 µm, inlet temperature 75°C, atomization air 0.15 MPa, drying air flow 0.4 m³/min and spray rate 22 g/min. The suspension was stirred throughout of process. After spray drying the SDD (Table 2) was spread on a tray and placed in a Fisher Scientific Istotemp Model 655F oven at 60°C for 60 minutes for a secondary drying step. The SDD was filled in HPMC capsules size 0. The final F5 capsules composition is shown in Table 7.

**Table 7. Composition of F5 capsules**

| **Ingredient** | **mg/capsule** |
|---|---|
| Compound 1 | 50 |
| Labrasol^{™} | 100 |
| Neusilin^{™} | 100 |
| **Total:** | **250** |

### (h) Solid dispersion in PVP with surfactant - Tablet formulation (F6(a) to F6(d))

The solid dispersion was first prepared following the procedure detailed in Example 1(d) using the weight proportions indicated in the first section of Table 8 (i.e. a drug load of about 40 wt.%) and a dichloromethane/methanol proportion of 33:67 as solvent. Tablets were manufactured by dry granulation (roller compaction) from the solid dispersion. The intragranular ingredients in Table 8 were sieved through a No. 30 sieve (600µm nominal aperture) and mixed using a V-blender for 2 min at 25 rpm (pre-mix). The intragranular lubricant was sieved through a No. 40 sieve (425µm nominal aperture) and added to the pre-mix for 2 min at 25 rpm. The lubricated mixture was roller compacted using a TFC-Labo^{™} roller compactor (Vector Corporation). Ribbons having thickness about 0.8 mm were obtained using at roll force of 750 psi, and roll/screw feeder speeds of 3.5 ± 0.5 / 40 ± 5 rpm, respectively. The ribbons were granulated using a No. 20 mesh sieve (850 µm nominal aperture). The resulting granules were transferred to the V-blender and mixed for 2 min at 25 rpm with the extra granular lubricant previously sieved through a No. 40 sieve. The tablets (F6(a) to F6(c)) were compressed with a KORSCH XP1^{™} one-station tablet press equipped with a gravity feeder and 7 mm, 8mm, round tooling for the 20mg and 35 mg tablets and 6.05 x 17.75 mm capsule shape tooling for the 100mg tablets, respectively. The target tablet weights were 114mg, 200mg, and 571mg for the 20mg, 35mg and 100 mg strengths, respectively. Hardness was adjusted to target disintegration below 30 minutes.

**Table 8. Composition of F6(a) to F6(c) tablets**

| **Ingredient** | **Function** | **mg/tablet** | | |
|---|---|---|---|---|
| | | **F6(a)** | **F6(b)** | **F6(c)** |
| Compound 1 | Solid dispersion | 35.0 | 20.0 | 100.0 |
| Povidone | | 48.2 | 27.5 | 137.5 |
| Sodium lauryl sulfate | | 4.4 | 2.5 | 12.5 |
| Microcrystalline cellulose | Excipients (intragranular) | 50.4 | 28.8 | 143.9 |
| Lactose | | 46.0 | 26.3 | 131.3 |
| Sodium lauryl sulfate | | 4.0 | 2.3 | 11.4 |
| Croscarmellose | | 10.0 | 5.7 | 28.6 |
| Magnesium stearate | | 1.5 | 0.83 | 4.28 |
| Magnesium stearate | Lubricant (extragranular) | 0.5 | 0.27 | 1.42 |
| **Total:** | | **200** | **114** | **571** |

An F6(d) formulation was also prepared by introducing formulation F6(b) into a capsule.

### (i) Solid dispersion in PVP with surfactant (F7)

The solid dispersion was first prepared following the procedure detailed in Example 1(d) by replacing sodium lauryl sulfate with Poloxamer^{™} and using the weight proportions indicated in Table 9.

**Table 9. Composition of F7 capsules**

| **Ingredient** | **mg/capsule** |
|---|---|
| Compound 1 | 22 |
| Povidone | 82.5 |
| Poloxamer^{™} | 5.5 |
| Microcrystalline cellulose | 22 |
| **Total:** | **132** |

### (j) Solid dispersion in HPMC-AS with surfactant (F8)

Hypromellose acetate succinate and sodium lauryl sulfate were first dissolved in dichloromethane/methanol (50:50 v/v). Compound 1 was added to the solution and dissolved. The solution was spray dried using a Yamato Lab Spray Dryer Model GB210 with internal nozzle diameter 711 µm, inlet temperature 65°C, atomization air 0.15 MPa, drying air flow 0.4 m³/min and spray rate 22 g/min. No secondary drying was needed. The SDD was filled in HPMC capsules size 0. The final F8 capsule composition is shown in Table 10.

**Table 10. Composition of F8 capsules**

| **Ingredient** | **mg/capsule** |
|---|---|
| Compound 1 | 15 |
| HPMC-AS | 56.3 |
| Sodium lauryl sulfate | 3.8 |
| **Total:** | **75** |

### (k) Solid dispersion in PVP with surfactant (F9 to F13)

The solid dispersions were obtained by spray-drying a solution containing Compound 1, povidone and sodium lauryl sulfate (4.8% by weight of dissolved solids, see first section of Table 11 for proportions) in a 1:1 mixture of dichloromethane and methanol using a Yamato Lab Spray Dryer Model GB22 with internal nozzle diameter 711 µm at inlet temperature 65°C, atomization air 0.15 MPa, drying air flow of 0.4 m³/min and spray rate 25 ± 2 g/min. To remove potential residual solvents, the spray dried materials were spread on a tray and submitted to a secondary drying at 60°C for 2 hours into a Fisher Scientific Istotemp Model 655F conventional oven to provide the SDD.

The final blends were manufactured at 40 grams batch size by dry granulation (roller compaction). The intragranular ingredients (i.e., SDD mixed with ingredients from second section of Table 11) were sieved through a No. 30 sieve (600 µm nominal aperture) and mixed using a V-blender for 2 minutes at 25 rpm (pre-mix). The intragranular lubricant was sieved through a No. 40 sieve (425 µm nominal aperture) and mixed with the pre-mix for 2 minutes at 25 rpm.

The lubricated mixture was then roller compacted using a TFC-Labo^{™} roller compactor (Vector Corporation). Ribbons having thickness about 1 mm were obtained using at roll force of 700 psi, and roll/screw feeder speeds of 3 and 38 rpm, respectively and they were granulated using a No. 20 mesh sieve (850 µm nominal aperture).

The resulting granules were transferred to the V-blender and mixed for 2 minutes at 25 rpm with the extragranular excipients (see last section of Table 11) previously sieved through a No. 40 sieve and lubricated by mixing for 2 minutes at 25 rpm with magnesium stearate previously sieved through a No. 40 sieve.

### Tablets

20-mg tablets with a drug load of 17.5% (SDD 40% DL) were compressed with a KORSCH XP1^{™} one-station tablet press equipped with 7 mm round standard tooling at tablet weight 114 ± 3mg and tablet hardness 4 ± 1kp.

20-mg tablets with a drug load of 26.3% (SDD 60% DL) were compressed with a KORSCH XP1^{™} one-station tablet press equipped with 6 mm round standard tooling at tablet weight 76 ± 2 mg and tablet hardness 3 ± 1 kp.

20-mg tablets with a drug load of 35.0% (SDD 80% DL) were compressed with a KORSCH XP1^{™} one-station tablet press equipped with 5 mm round standard tooling at tablet weight 57 ± 1 mg and tablet hardness 3 ± 1 kp.

Summary of drug product composition is listed in Table 11 below.

**Table 11. Composition of F9 to F13 tablets**

| **Ingredient** | **mg/tablet** | | | | |
|---|---|---|---|---|---|
| | **F9** | **F10** | **F11** | **F12** | **F13** |
| Compound 1 | 20.0 | 20.0 | 20.0 | 20.0 | 20.0 |
| Povidone | 29.9 | 27.5 | 26.0 | 11.7 | 3.7 |
| Sodium lauryl sulfate | 1.0 | 2.5 | 4.0 | 1.7 | 1.3 |
| Microcrystalline cellulose | 28.5 | 27.0 | 28.5 | 19.0 | 14.3 |
| Lactose monohydrate | 26.4 | 26.2 | 26.4 | 17.6 | 13.2 |
| Sodium lauryl sulfate | 2.3 | 2.3 | 2.3 | 1.5 | 1.1 |
| Croscarmellose sodium | 2.9 | 2.9 | 2.9 | 1.9 | 1.4 |
| Magnesium stearate | 0.9 | 0.9 | 0.9 | 0.6 | 0.4 |
| Sodium lauryl sulfate | -- | 1.7 | -- | -- | -- |
| Croscarmellose sodium | 2.9 | 2.9 | 2.9 | 1.9 | 1.4 |
| Magnesium stearate | 0.3 | 0.3 | 0.3 | 0.2 | 0.1 |
| **Total:** | **114** | **114** | **114** | **76** | **57** |

### (I) Solid dispersion in HPMC-AS (F14)

The solid dispersion of Compound 7 in HPMC-AS was obtained by spray-drying using a Yamato Lab Spray Dryer Model GB22 with 711 µm internal nozzle diameter. The solution (5% solids) in acetone was spray-dried at an inlet temperature of 60-65°C, atomization air at 0.15 MPa, drying air flow at 0.4 m³/min, and spray rate of 20 g/min. To remove potential residual solvents, the spray dried material also underwent a secondary drying in a Fisher Scientific Isotemp model 280A vacuum oven at 60°C and - 18 in Hg between 15 minutes and 1 hour.

The formulation was filled manually into appropriately sized hard gelatin capsules for a 20mg Compound 7 capsule. No other ingredients were added. The final drug product formulation is presented in Table 12. The total weight excludes the weight of the capsule.

**Table 12. Composition of F14 capsules**

| **Ingredient** | **mg/capsule** |
|---|---|
| Compound 7 | 20.0 |
| HPMC-AS | 91.1 |
| **Total:** | **111.1 mg** |

### (m) Solid dispersion in HPMC-AS (F15(a) and F15(b))

The solid dispersion of Compound 7 in HPMC-AS was obtained by spray-drying using a Yamato Lab Spray Dryer Model GB22 with 711 µm internal nozzle diameter. The solution (5% solids) in a 1:1 mixture of dichloromethane and methanol was spray-dried at an inlet temperature of 60-65°C, atomization air at 0.15 MPa, drying air flow at 0.4 m³/min, and spray rate of 20 g/min. To remove potential residual solvents, the spray dried material also underwent a secondary drying in a Fisher Scientific Isotemp 280A vacuum oven at 60°C and - 18 in Hg between 15 minutes and 1 hour.

The formulation was processed using dry granulation. The solid dispersion and powders except lubricant (magnesium stearate) and dibasic calcium phosphate were screened with 30 mesh (600 µm) sieve and mixed using a V-blender at 25 rpm for 2 minutes. The intragranular magnesium stearate was screened through a 40 mesh (425 µm) sieve and mixed with unlubricated pre-mix using a V-blender at 25 rpm for 2 minutes. The blend was roller compacted using a TFC-Lab^{™} roller compactor (Vector Corporation). Ribbons having a thickness about 1 mm were obtained using at roll force of 700 psi, roll speed 2.5±0.5 rpm, and screw feeder speed 25±5 rpm. The ribbons were granulated using a 20 mesh (850 µm) sieve. Granules and external phase disintegrant (croscarmellose) and lubricant (magnesium stearate) were mixed 2 minutes each using a V-blender at 25 rpm.

The 5 and 50 mg tablets were compressed using the Korsch^{™} XP1 equipped with a gravity feeder and with 5 and 12 mm round standard concave tooling, respectively. Composition of the tablets is summarized in Table 13.

**Table 13. Composition of F15(a) and F15(b) tablets**

| **Ingredient** | **Function** | **mg/tablet** | |
|---|---|---|---|
| | | **F15(a)** | **F15(b)** |
| Compound 7 | Solid dispersion | 5.0 | 50.0 |
| HMPC-AS | | 7.5 | 75.1 |
| Microcrystalline cellulose | Excipients (intragranular) | 20.4 | 204.0 |
| Dibasic calcium phosphate anhydrous | | 21.1 | 210.9 |
| Meglumine | | 1.2 | 12.0 |
| Sodium lauryl sulfate | | 1.2 | 12.0 |
| Croscarmellose | | 1.5 | 15.0 |
| Magnesium stearate | | 0.45 | 4.5 |
| Croscarmellose | Excipients (extragranular) | 1.5 | 15.0 |
| Magnesium stearate | | 0.15 | 1.5 |
| **Total:** | | **60** | **600** |

### (n) Solid dispersion in PVP with surfactant (F16(a) and F16(b))

The solid dispersion of Compound 7 in PVP and sodium lauryl sulfate was obtained by spray-drying using a Yamato Lab Spray Dryer Model GB22 with 711 µm internal nozzle diameter. The solution (5% solids) in a 1:1 mixture of dichloromethane and methanol was spray-dried at an inlet temperature of 60-65°C, atomization air at 0.15 MPa, drying air flow at 0.4 m³/min, and spray rate of 20 g/min. To remove potential residual solvents, the spray dried material also underwent a secondary drying in a Fisher Scientific Isotemp model 280A vacuum oven at 60°C and - 18 in Hg between 15 minutes and 1 hour.

### 20mg tablets:

The 20mg dosage form was processed using dry granulation. The powders except lubricant (magnesium stearate) were screened with 30 mesh (600 µm) sieve and mixed using a V-blender at 25 rpm for 2 minutes. The intragranular magnesium stearate was screened through a 40 mesh (425 µm) sieve and mixed with unlubricated pre-mix using a V-blender at 25 rpm for 2 minutes. The blend was roller compacted using a TFC-Lab^{™} roller compactor (Vector Corporation). Ribbons having a thickness about 1 mm were obtained using at roll force of 700 psi, roll speed 3.5 ± 0.5 rpm, and screw feeder speed 45 ± 5 rpm. The ribbons were granulated using a 20 mesh (850 µm) sieve. Granules and external phase lubricant were mixed using a V-blender at 25 rpm for 2 minutes. The 20 mg Compound 7 tablets were compressed using the Korsch^{™} XP 1 equipped with a gravity feeder and 7 mm round standard concave tooling.

### 100 mg tablets:

The 100 mg tablets were made using the same SDD as for the 20mg tablets but instead of dry roller compaction, a slugging method was used. The ingredients except for lubricant were screened with a 30-mesh sieve and mixed using a mortar/pestle for 2 minutes. The slugs having weight 352 ± 2 mg, thickness 5±1 mm, and hardness 1.8 ± 0.2 kp were compressed using a Carver manual hydraulic bench top lab press at 500 lbf. The slugs were crushed and granulated thought an 850 µm sieve. The granules were lubricated by mixing with external lubricant for 1 minutes using a mortar/pestle. The 100 mg Compound 7 tablets were compressed using the Korsch^{™} XP 1 equipped with a gravity feeder.

Table 14 summarizes the composition for the 20 mg and 100 mg F16(a) and F16(b) tablets.

**Table 14. Composition of F16(a) and F16(b) tablets**

| **Ingredient** | **Function** | **mg/tablet** | |
|---|---|---|---|
| | | **F16(a)** | **F16(b)** |
| Compound 7 | Solid dispersion | 20.0 | 100.0 |
| Povidone | | 27.5 | 137.5 |
| Sodium lauryl sulfate | | 2.5 | 12.5 |
| Microcrystalline cellulose | | 26.7 | 133.3 |
| Lactose monohydrate | Internal phase excipients | 25.6 | 127.8 |
| Sodium lauryl sulfate | | 2.2 | 11.1 |
| Croscarmellose sodium | | 5.6 | 27.8 |
| Magnesium stearate | | 0.8 | 4.2 |
| Magnesium stearate | External phase excipient | 0.3 | 1.4 |
| **Total:** | | **111.1** | **555.6** |

### (o) Solid dispersion in PVP with surfactant (F17(a) and F17(b))

The solid dispersion of Compound 7 in PVP and sodium lauryl sulfate was obtained as in 1(n) using the weight proportions described in Table 15 below.

A final blend was then prepared using a dry granulation process. The powders except lubricant (magnesium stearate) were screened with 30 mesh (600 µm) sieve and mixed using a V-blender at 25 rpm for 2 minutes. The intragranular magnesium stearate was screened through a 40 mesh (425 µm) sieve and mixed with unlubricated pre-mix using a V-blender at 25 rpm for 2 minutes. The blend was roller compacted using a TFC-Lab^{™} roller compactor (Vector Corporation). Ribbons having a thickness about 1 mm were obtained using at roll force of 700 psi, roll speed 2.5 ± 0.5 rpm, and screw feeder speed 25 ± 5 rpm. The ribbons were granulated using a 20 mesh (850 µm) sieve. Granules and external phase lubricant (magnesium stearate) were mixed 2 minutes each using a V-blender at 25 rpm.

The 5 and 50 mg tablets were then compressed using the Korsch^{™} XP1 equipped with a gravity feeder and with 5 and 12 mm round standard concave tooling, respectively.

The final F17(a) and F17(b) formulations are presented in Table 15 below.

**Table 15. Composition of F17(a) and F17(b) tablets**

| **Ingredient** | **Function** | **mg/tablet** | |
|---|---|---|---|
| | | **F17(a)** | **F17(b)** |
| Compound 7 | Solid dispersion | 5.0 | 50.0 |
| Povidone | | 6.9 | 68.8 |
| Sodium lauryl sulfate | | 0.6 | 6.3 |
| Microcrystalline cellulose | Internal phase excipients | 21.0 | 210.0 |
| Lactose monohydrate | | 21.7 | 216.9 |
| Sodium lauryl sulfate | | 1.2 | 12.0 |
| Croscarmellose | | 3.0 | 30.0 |
| Magnesium stearate | | 0.45 | 4.5 |
| Magnesium stearate | External phase excipient | 0.15 | 1.5 |
| **Total:** | | **60** | **600** |

### (p) Solid dispersion in PVP with surfactant (F18(a) and F18(b))

The F18(a) and F18(b) formulations were prepares as in 1(o) where lactose is replaced with anhydrous dibasic calcium phosphate and meglumine in the intragranular ingredients and croscarmellose is also present in the external phase. The granulation process is the same as the one presented in 1(o). The final formulation is provided in Table 16.

**Table 16. Composition of F18(a) and F18(b) tablets**

| **Ingredient** | **Function** | **mg/tablet** | |
|---|---|---|---|
| | | **F18(a)** | **F18(b)** |
| Compound 7 | Solid dispersion | 5.0 | 50.0 |
| Povidone | | 6.9 | 68.8 |
| Sodium lauryl sulfate | | 0.6 | 6.3 |
| Microcrystalline cellulose | Internal phase excipients | 20.4 | 204.0 |
| Dibasic calcium phosphate anhydrous | | 21.1 | 210.9 |
| Meglumine | | 1.2 | 12.0 |
| Sodium lauryl sulfate | | 1.2 | 12.0 |
| Croscarmellose | | 1.5 | 15.0 |
| Magnesium stearate | | 0.45 | 4.5 |
| Magnesium stearate | External phase excipients | 0.15 | 1.5 |
| Croscarmellose | | 1.5 | 15.0 |
| **Total:** | | **60** | **600** |

### (q) Solid dispersion in PVP with surfactant (F19(a) and F19(b))

The F19(a) and F19(b) formulations were prepares as in 1(p) where sodium bicarbonate is added to the intragranular ingredients. The granulation process is the same as the one presented in 1(o). The final formulation is provided in Table 17.

**Table 17. Composition of F19(a) and F19(b) tablets**

| **Ingredient** | **Function** | **mg/tablet** | |
|---|---|---|---|
| | | **F19(a)** | **F19(b)** |
| Compound 7 | Solid dispersion | 5.0 | 50.0 |
| Povidone | | 6.9 | 68.8 |
| Sodium lauryl sulfate | | 0.6 | 6.3 |
| Microcrystalline cellulose | Internal phase excipients | 20.4 | 204.0 |
| Lactose monohydrate | | 21.1 | 210.9 |
| Sodium bicarbonate | | 1.2 | 12.0 |
| Sodium lauryl sulfate | | 1.2 | 12.0 |
| Croscarmellose | | 1.5 | 15.0 |
| Magnesium stearate | | 0.45 | 4.5 |
| Magnesium stearate | External phase excipients | 0.15 | 1.5 |
| Croscarmellose | | 1.5 | 15.0 |
| **Total:** | | **60** | **600** |

### Example 2 - Physicochemical properties

Crystal state was verified by X-ray powder diffraction (XRPD). The powder was analyzed using a low volume sample holder (Si low background sample holder (Ø 51.5 mm) with Ø 20 mm x 0.5 mm sample cavity) kept under a constant rotation of 15 rpm during the analysis using a Bruker D2 Phaser X-ray diffractometer with LynxEye detector, Cu Kα radiation (λ=1.5406 Å). Acquisition was done over a range of 3-56° 2θ, increment of 0.01°2θ with 0.2 second step time using a 0.6 mm opening slit with a 2.5 mm detector window.

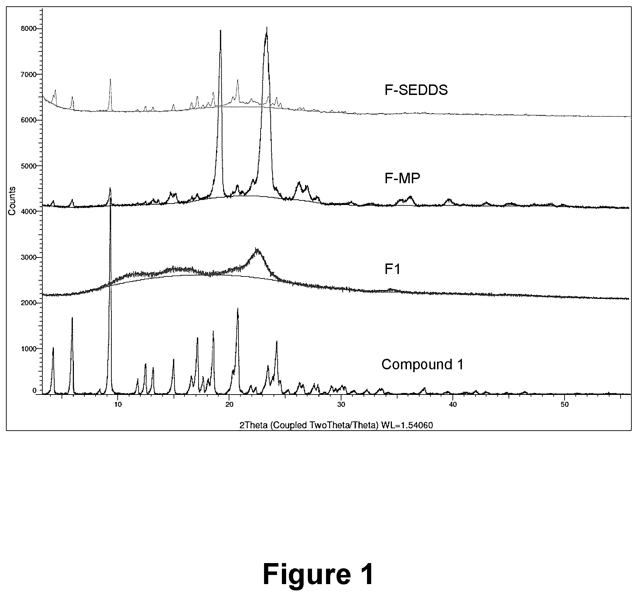

The XRPD pattern was first measured for the spray dried formulation F1 (without surfactant) and compared with XRPD patterns of solid dispersions prepared by other methods (F-MP and F-SEDDS) and with Compound 1 as a reference. The XRPD patterns are shown in Figure 1. The results show that a complete amorphous conversion of Compound 1 was only achieved with the high glass transition temperature polymer (in F1) while the melted polymer and S-SEDDS formulations showed residual peaks from Compound 1.

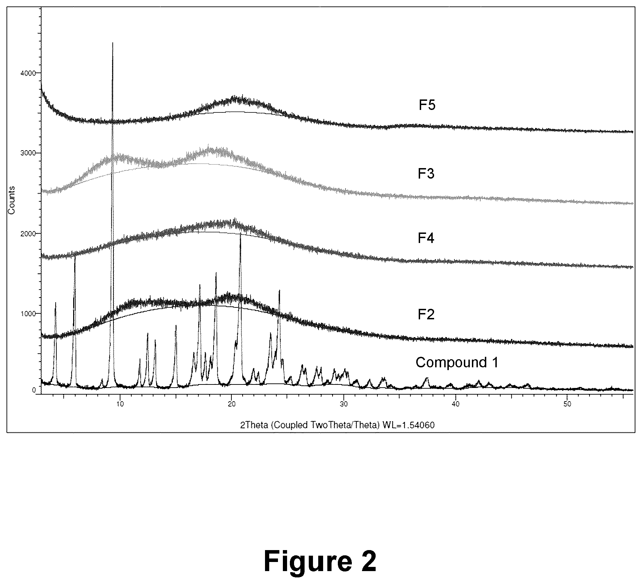

XRPD patterns were also measured for formulations F2 to F5 and compared to Compound 1 as shown in Figure 2. Amorphous form was confirmed for all four formulations.

### Example 3 - Biological properties

Pharmacokinetic properties were measured in Beagle dogs. Administration was done orally and compared with intravenous administration (2 mg/kg, in DMSO/tetraglycol/PEG400/water, 10:20:25:45). Each formulation was tested as a single dose on three (3) animals except for F6(b), which was tested on five (5) animals. Dogs were sampled at times 0, 1h, 4h, 8h, 12h, 24h, 36h, 48h, and 72h to give full time-concentration profiles. All tests were carried out with fasted dogs except test on normal dogs using F-SEDDS, which was carried on fed dogs. Testing conditions and oral bioavailability results and summarized in Tables 18 and 19. The results are as presented as an average between the tested animals.

**Table 18. Pharmacokinetic results for Compound 1 formulations**

| **Formulation** | **Dogs cohort^{a}** | **Dose (mg/tablet)** | **Dose (mg/kg)** | **Tₘₐₓ (h)** | **Cmax (ng/mL)** | **AUC_{inf} (ng/mL*h)** | **T_{1/2} (h)** | **Oral F%** |
|---|---|---|---|---|---|---|---|---|
| i.v. | Small | N/A | 2 | N/A | C₀ 1823 | 9100 | 6.26 | N/A |
| i.v. | Normal | N/A | 2 | N/A | C₀ 2221 | 15,417 | 5.88 | N/A |
| F-MP | Small | 50 | 6.7 | 3 | 23.8 | 779 | 41.3 | 2.6 |
| F-SEDDS | Small | 50 | 6.7 | 2 | 3.4 | 133 | 33 | 0.4 |
| F-SEDDS | Normal | 50 | 4.5 | 5.7 | 27 | 680 | 26 | 2 |
| F1 | Small | 50 | 6.7 | 4 | 209 | 2755 | 24.4 | 9.2 |
| F2(a) | Normal | 50 | 4.5 | 4 | 1270 | 20,811 | 9.8 | 59.9 |
| F2(b) | Normal | 22 | 2 | 4 | 594 | 8298 | 18.4 | 51.7 |
| F2(c) | Small | 35 | 4.7 | 1 | 728 | 9368 | 16.1 | 45.2 |
| F2(d) | Small | 150 | 20 | 2 | 2010 | 30,057 | 13.3 | 38.8 |
| F3 | Small | 50 | 6.7 | 3 | 412 | 6266 | 25.1 | 20.9 |
| F4 | Normal | 50 | 6.7 | 2.0 | 1501 | 23,893 | 9.7 | 68.7 |
| F5 | Normal | 50 | 6.7 | 9.3 | 126 | 2572 | 35.5 | 12.6 |
| F6(a) | Small | 35 | 4.7 | 1 | 923 | 11,039 | 12.6 | 55.7 |
| F6(b) | Small | 20 | 2.2 | 3.8 | 563 | 6890 | 9.7 | 65.9 |
| F6(d) | Small | 20 | 2.3 | 1 | 493 | 5,015 | 6.9 | 45.1 |
| F7 | Normal | 22 | 2 | 9.7 | 84.9 | 2002 | 18.4 | 12.9 |
| F8 | Small | 15 | 2 | 3 | 322 | 4778 | 27.4 | 51 |
| F9 | Small | 20 | 2.4 | 1.0 | 181 | 2588 | 6.3 | 23.1 |
| F10 | Small | 20 | 2.5 | 1.0 | 369 | 4719 | 8.1 | 41.5 |
| F11 | Small | 20 | 2.5 | 3.0 | 321 | 3621 | 7.3 | 17.2 |
| F12 | Small | 20 | 2.5 | 12.3 | 232 | 3737 | 6.7 | 29.8 |
| F13 | Small | 20 | 2.5 | 1.0 | 47.3 | 795 | 10.2 | 4.4 |

| | | | | | | | | |
|---|---|---|---|---|---|---|---|---|
| a. Small: average body weight of about 7.5 kg; Normal: average body weight of about 11 kg | | | | | | | | |

**Table 19. Pharmacokinetic results for Compound 7 formulations**

| **Formulation** | **Dogs mean BW^{a}** | **Dose (mg/tablet)** | **Dose (mg/kg)** | **Tₘₐₓ (h)** | **Cₘₐₓ (ng/mL)** | **AUC_{inf} (ng/mL*h)** | **T_{1/2} (h)** | **Oral F%** |
|---|---|---|---|---|---|---|---|---|
| F14 | 8.72 kg | 20 | 2.3 | 3.3 | 449 | 16599 | 29.0 | 61.3 |
| F16(a) | 8.41 kg | 20 | 2.4 | 2.7 | 369 | 11134. | 20.0 | 40.1 |
| F16(b) | 8.17 kg | 100 | 12.24 | 4.0 | 681 | 19196 | 18.5 | 11.1 |

| | | | | | | | | |
|---|---|---|---|---|---|---|---|---|
| a. BW= body weight | | | | | | | | |

Other formulations tested included wet granulation, melt granulation, and dry granulation with or without an enteric coating. Oral bioavailability observed for these formulations ranged from 2% to 5%. As can be seen from Tables 18, high T_{g} polymer matrices without surfactant such as in F1 and F3 resulted in a significant improvement in bioavailability compared to the melted polymer (F-MP) and solid self-emulsifying drug delivery system (F-SEDDS). Furthermore, data also suggest that surfactant containing polymer matrices result in improved bioavailability compared to the counterpart containing an inorganic Neusilin matrix (F5). Highest improvements in bioavailability results were generally obtained with a drug load of about 20% to about 60% (w/w) in the solid spray dried dispersion along with a concentration of around 5% by weight of a surfactant such as sodium lauryl sulfate.

Stability tests were also performed on the present formulations. Some degradation (around 1%) was observed, for instance, with formulations containing HPMC-AS as a polymer in accelerated degradation 1-month tests. Formulations containing PVP and SLS were found to be generally more stable and under testing conditions.

## Claims

1. A solid dispersion comprising a compound dispersed in a solid matrix comprising a pharmaceutically acceptable polymer having a glass transition temperature of at least 50°C, wherein the compound is selected from: 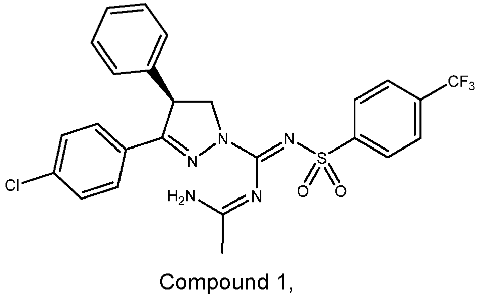 or 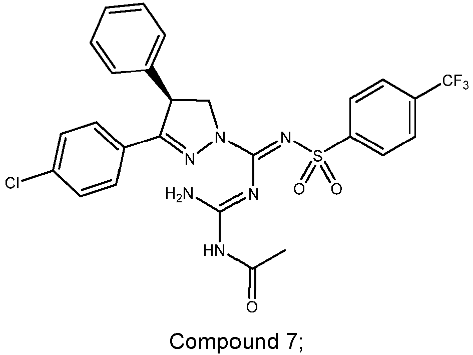
or a tautomer or a pharmaceutically acceptable salt thereof;
wherein the compound is in a substantially amorphous form;
wherein the polymer:compound weight ratio is within the range of 1:1 to 6:1, or preferably 1:1 to 4:1, or preferably 2:1 to 5:1, or preferably 3:1 to 5:1, or preferably 1:1 to 3:1, and
wherein the compound concentration in the solid dispersion is within the range of 15% to 60% by weight, preferably 18% to 40% by weight, or preferably within the range of 20% to 40% by weight, or preferably within the range of 30% to 50% by weight.

2. The solid dispersion of claim 1, having a polymer:compound weight ratio within the range of 1:1 to 4:1, or 2:1 to 5:1, or 3:1 to 5:1, or 1:1 to 3:1.

3. The solid dispersion of claim 1 or claim 2, wherein the compound concentration in the solid dispersion is within the range of 18% to 40% by weight, or within the range of 20% to 40% by weight, or within the range of 30% to 50% by weight.

4. The solid dispersion of any one of claims 1 to 3, wherein said polymer has a glass transition temperature of at least 80°C, or at least 100°C, or at least 120°C, or at least 140°C, or between 50°C and 200°C, or between 80°C and 200°C, or between 100°C and 180°C.

5. The solid dispersion of any one of claims 1 to 4, wherein said polymer is:
- a polyvinylpyrrolidone or a copolymer thereof; or
- cellulose or a cellulose derivative, preferably a cellulose derivative which is an esterified hydroxyalkyl methylcellulose; or
- a polyethylene glycol, polylactic acid or polymethacrylate.

6. The solid dispersion of any one of claims 1 to 5, wherein said polymer is polyvinylpyrrolidone having an average molecular weight of between 5,000 and 100,000, or is hydroxypropyl methylcellulose acetate succinate.

7. The solid dispersion of any one of claims 1 to 6, further comprising a pharmaceutically acceptable surfactant, wherein said pharmaceutically acceptable surfactant comprises at least one surfactant selected from:
- long chain alkyl or alkenyl sulfate salts;
- alkyl sulfonate salts;
- sorbitan long chain carboxylic acid esters;
- pegylated sorbitan long chain carboxylic acid esters;
- polyethylene-polypropylene glycol block copolymers;
- pegylated or non-pegylated mono, di, and tri glyceride long chain carboxylic acid;
- polyethylene and/or polypropylene glycol alkoxylates;
- alkylphenol alkoxylates;
- alkylphenol derivatives of polyethylene and/or polypropylene glycols; and
- sucrose long chain carboxylic acid esters.

8. The solid dispersion of claim 7, wherein the pharmaceutically acceptable surfactant is a long chain alkyl sulfate, preferably sodium lauryl sulfate.

9. The solid dispersion of claim 7 or claim 8, having a polymer:surfactant weight ratio within the range of 5:1 to 20:1, or of 10:1 to 15:1.

10. The solid dispersion of any one of claims 1 to 9, in powder form or in particulate form.

11. A process for the preparation of a solid dispersion as defined in any one of claims 1 to 10, comprising a step of mixing the compound and the polymer, preferably said mixing comprises the steps of:
(a) dissolving the compound and polymer in a solvent;
(b) drying the mixture obtained in (a).

12. The process of claim 11, wherein said drying is carried out by spray drying, the mixing preferably being carried out by rapid acoustic mixing, extrusion, planetary mixing and ball milling.

13. A solid oral pharmaceutical composition comprising a solid dispersion as defined in any one of claims 1 to 10.

14. The solid oral pharmaceutical composition of claim 13, further comprising a pharmaceutically acceptable carrier, diluent, or excipient.

15. The solid oral pharmaceutical composition of claim 14, wherein said carrier, diluent or excipient is a binder selected from cellulose-based substances such as microcrystalline cellulose and carboxymethylcellulose, and other binders like gum acacia, gelatin, corn starch, gum tragacanth, sodium alginate, lactose, sorbitol, mannitol, dextrose, kaolin, cellulose, calcium carbonate, calcium silicate, dicalcium phosphate, and polyethylene glycol (PEG), or a combination thereof, preferably said binder is microcrystalline cellulose.

16. The solid oral pharmaceutical composition of claim 14, wherein said carrier, diluent or excipient is a diluent selected from lactose, sorbitol, mannitol, dextrose, kaolin, cellulose, calcium carbonate, calcium silicate and dicalcium phosphate.

17. The solid oral pharmaceutical composition of any one of claims 13 or 24, wherein the compound is present in said composition at a concentration of between 5 wt.% and 50 wt.%, or between 10 wt.% and 40 wt.%.

18. The solid oral pharmaceutical composition of any one of claims 13 to 17, in the form of a tablet or capsule.

19. The solid oral pharmaceutical composition of any one of claims 13 to 18, in unit dosage form comprising the compound in an amount within the range of 20 to 200 mg per dose.

20. A solid dispersion as defined in any one of claims 1 to 10 or a solid oral pharmaceutical composition as defined in any one of claims 13 to 19 for use in the treatment of a disease or disorder selected from diabetes (type 1 or 2), obesity, non-alcoholic and alcoholic fatty liver disease (a risk factor for insulin resistance), a co-morbidity of obesity, a co-morbidity of diabetes, Prader-Willi Syndrome (PWS), Pro-opiomelanocortin (POMC) deficiency obesity, LepR deficiency obesity, POMC heterozygous deficiency obesity, POMC epigenetic disorders, Bardet-Biedl syndrome, Alström syndrome, dyslipidemia predisposing to arteriosclerotic heart disease, diabetic nephropathy, fibrosis and fibrotic diseases such as Idiopathic Pulmonary Fibrosis (IPF) and Hermansky-Pudlak Syndrome pulmonary fibrosis (HPS-PF), and gout.

21. The solid dispersion or solid oral pharmaceutical composition for use of claim 20, wherein the co-morbidity of obesity is selected from metabolic syndrome, dementia, heart disease, hypertension, gallbladder disease, gastrointestinal disorders, menstrual irregularities, degenerative arthritis, venous statis ulcer, pulmonary hypoventilation syndrome, sleep apnea, snoring, coronary artery disease, arterial sclerotic disease, pseudotumor cerebri, osteoarthritis, high cholesterol, and increased incidence of malignancies of the liver, ovaries, cervix, uterus, breasts, prostate, or gallbladder.

22. The solid dispersion or solid oral pharmaceutical composition for use of claim 20, wherein the co-morbidity of diabetes is selected from diabetic nephropathy, chronic kidney disease, diabetic retinopathy, and peripheral and autonomic neuropathy.

## Patentansprüche

1. Feste Dispersion, umfassend eine in einer festen Matrix dispergierte Verbindung, umfassend ein pharmazeutisch akzeptables Polymer mit einer Glasübergangstemperatur von mindestens 50 °C, wobei die Verbindung ausgewählt ist aus: 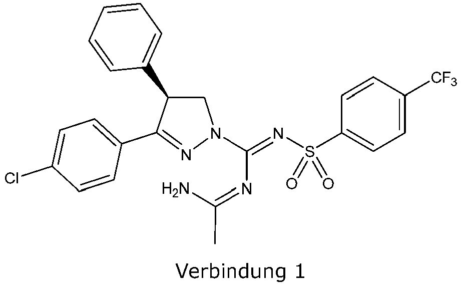 oder 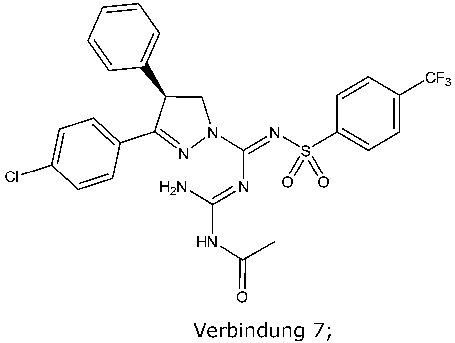
oder ein Tautomer oder ein pharmazeutisch akzeptables Salz davon;
wobei die Verbindung in einer im Wesentlichen amorphen Form vorliegt;
wobei das Gewichtsverhältnis Polymer:Verbindung im Bereich von 1:1 bis 6:1 oder vorzugsweise 1:1 bis 4:1 oder vorzugsweise 2:1 bis 5:1 oder vorzugsweise 3:1 bis 5:1 oder vorzugsweise 1:1 bis 3:1 liegt und
wobei die Konzentration der Verbindung in der festen Dispersion im Bereich von 15 bis 60 Gew.-%, vorzugsweise 18 bis 40 Gew.-%, oder vorzugsweise im Bereich von 20 bis 40 Gew.-%, oder vorzugsweise im Bereich von 30 bis 50 Gew.-% liegt.

2. Feste Dispersion nach Anspruch 1 mit einem Gewichtsverhältnis Polymer:Verbindung im Bereich von 1:1 bis 4:1 oder 2:1 bis 5:1 oder 3:1 bis 5:1 oder 1:1 bis 3:1.

3. Feste Dispersion nach Anspruch 1 oder Anspruch 2, wobei die Konzentration der Verbindung in der festen Dispersion im Bereich von 18 bis 40 Gew.-% oder im Bereich von 20 bis 40 Gew.-% oder im Bereich von 30 bis 50 Gew.-% liegt.

4. Feste Dispersion nach einem der Ansprüche 1 bis 3, wobei das Polymer eine Glasübergangstemperatur von mindestens 80 °C oder mindestens 100 °C oder mindestens 120 °C oder mindestens 140 °C oder zwischen 50 °C und 200 °C oder zwischen 80 °C und 200 °C oder zwischen 100 °C und 180 °C aufweist.

5. Feste Dispersion nach einem der Ansprüche 1 bis 4, wobei das Polymer:
- ein Polyvinylpyrrolidon oder ein Copolymer davon; oder
- Cellulose oder ein Cellulosederivat, vorzugsweise ein Cellulosederivat, das eine veresterte Hydroxyalkylmethylcellulose ist; oder
- ein Polyethylenglykol, Polymilchsäure oder Polymethacrylat ist.

6. Feste Dispersion nach einem der Ansprüche 1 bis 5, wobei das Polymer Polyvinylpyrrolidon mit einem durchschnittlichen Molekulargewicht zwischen 5.000 und 100.000 oder Hydroxypropylmethylcelluloseacetatsuccinat ist.

7. Feste Dispersion nach einem der Ansprüche 1 bis 6, ferner umfassend ein pharmazeutisch akzeptables Tensid, wobei das pharmazeutisch akzeptable Tensid mindestens ein Tensid umfasst, das ausgewählt ist aus:
- langkettigen Alkyl- oder Alkenylsulfatsalzen;
- Alkylsulfonatsalzen;
- Sorbitanester langkettiger Carbonsäuren;
- pegylierten Sorbitanestern langkettiger Carbonsäuren;
- Polyethylen-Polypropylenglykol-Blockcopolymeren;
- pegylierter oder nicht pegylierter langkettiger Mono-, Di- und Triglyceridcarbonsäure;
- Polyethylen- und/oder Polypropylenglykolalkoxylaten;
- Alkylphenolalkoxylaten;
- Alkylphenolderivaten von Polyethylen- und/oder Polypropylenglykolen; und
- Saccharoseester langkettiger Carbonsäuren.

8. Feste Dispersion nach Anspruch 7, wobei das pharmazeutisch akzeptable Tensid ein langkettiges Alkylsulfat, vorzugsweise Natriumlaurylsulfat, ist.

9. Feste Dispersion nach Anspruch 7 oder Anspruch 8 mit einem Gewichtsverhältnis Polymer:Tensid im Bereich von 5:1 bis 20:1 oder von 10:1 bis 15:1.

10. Feste Dispersion nach einem der Ansprüche 1 bis 9 in Pulverform oder in Teilchenform.

11. Verfahren zur Herstellung einer festen Dispersion, wie in einem der Ansprüche 1 bis 10 definiert, umfassend einen Schritt des Mischens der Verbindung und des Polymers, wobei das Mischen vorzugsweise die Schritte umfasst:
(a) Auflösen der Verbindung und des Polymers in einem Lösungsmittel;
(b) Trocknen der in (a) erhaltenen Mischung.

12. Verfahren nach Anspruch 11, wobei das Trocknen durch Sprühtrocknen durchgeführt wird, wobei das Mischen vorzugsweise durch schnelles akustisches Mischen, Extrudieren, Planetenmischen und Kugelmahlen durchgeführt wird.

13. Feste orale pharmazeutische Zusammensetzung, umfassend eine feste Dispersion, wie in einem der Ansprüche 1 bis 10 definiert.

14. Feste orale pharmazeutische Zusammensetzung nach Anspruch 13, ferner umfassend einen pharmazeutisch akzeptablen Träger, ein Verdünnungsmittel oder einen Hilfsstoff.

15. Feste orale pharmazeutische Zusammensetzung nach Anspruch 14, wobei der Träger, das Verdünnungsmittel oder der Hilfsstoff ein Bindemittel ist, ausgewählt aus Substanzen auf Cellulosebasis wie mikrokristalliner Cellulose und Carboxymethylcellulose sowie anderen Bindemitteln wie Akaziengummi, Gelatine, Maisstärke, Tragantgummi, Natriumalginat, Lactose, Sorbit, Mannit, Dextrose, Kaolin, Cellulose, Calciumcarbonat, Calciumsilicat, Dicalciumphosphat und Polyethylenglykol (PEG) oder einer Kombination davon, wobei das Bindemittel vorzugsweise mikrokristalline Cellulose ist.

16. Feste orale pharmazeutische Zusammensetzung nach Anspruch 14, wobei der Träger, das Verdünnungsmittel oder der Hilfsstoff ein Verdünnungsmittel ist, ausgewählt aus Lactose, Sorbit, Mannit, Dextrose, Kaolin, Cellulose, Calciumcarbonat, Calciumsilicat und Dicalciumphosphat.

17. Feste orale pharmazeutische Zusammensetzung nach einem der Ansprüche 13 oder 24, wobei die Verbindung in der Zusammensetzung in einer Konzentration zwischen 5 Gew.-% und 50 Gew.-% oder zwischen 10 Gew.-% und 40 Gew.-% vorliegt.

18. Feste orale pharmazeutische Zusammensetzung nach einem der Ansprüche 13 bis 17 in Form einer Tablette oder Kapsel.

19. Feste orale pharmazeutische Zusammensetzung nach einem der Ansprüche 13 bis 18 in Einheitsdosierungsform, umfassend die Verbindung in einer Menge im Bereich von 20 bis 200 mg pro Dosis.

20. Feste Dispersion, wie in einem der Ansprüche 1 bis 10 definiert, oder feste orale pharmazeutische Zusammensetzung, wie in einem der Ansprüche 13 bis 19 definiert, zur Verwendung bei der Behandlung einer Krankheit oder Störung, ausgewählt aus Diabetes (Typ 1 oder 2), Adipositas, nichtalkoholischer und alkoholischer Fettlebererkrankung (ein Risikofaktor für Insulinresistenz), einer Komorbidität der Adipositas, einer Komorbidität des Diabetes, Prader-Willi-Syndrom (PWS), Proopiomelanocortin(POMC)-Mangel-Adipositas, LepR-Mangel-Adipositas, POMCheterozygoter-Mangel-Adipositas, POMC-epigenetischen Störungen, Bardet-Biedl-Syndrom, Alström-Syndrom, Dyslipidämie, die für arteriosklerotische Herzerkrankungen prädisponiert, diabetischer Nephropathie, Fibrose und fibrotischen Erkrankungen wie idiopathischer pulmonaler Fibrose (IPF) und pulmonaler Fibrose beim Hermansky-Pudlak-Syndrom (HPS-PF) sowie Gicht.

21. Feste Dispersion oder feste orale pharmazeutische Zusammensetzung zur Verwendung nach Anspruch 20, wobei die Komorbidität der Adipositas ausgewählt ist aus metabolischem Syndrom, Demenz, Herzerkrankungen, Hypertonie, Gallenblasenerkrankungen, gastrointestinalen Störungen, Menstruationsunregelmäßigkeiten, degenerativer Arthritis, venösem Stauungsulkus, pulmonalem Hypoventilationssyndrom, Schlafapnoe, Schnarchen, koronarer Herzkrankheit, arteriosklerotischer Erkrankung, Pseudotumor cerebri, Osteoarthritis, hohem Cholesterinspiegel und erhöhter Inzidenz von Malignomen der Leber, Eierstöcke, des Gebärmutterhalses, der Gebärmutter, der Brüste, der Prostata oder der Gallenblase.

22. Feste Dispersion oder feste orale pharmazeutische Zusammensetzung zur Verwendung nach Anspruch 20, wobei die Komorbidität des Diabetes ausgewählt ist aus diabetischer Nephropathie, chronischer Nierenerkrankung, diabetischer Retinopathie und peripherer und autonomer Neuropathie.

## Revendications

1. Dispersion solide comprenant un composé dispersé dans une matrice solide comprenant un polymère pharmaceutiquement acceptable ayant une température de transition vitreuse d'au moins 50 °C, dans laquelle le composé est choisi parmi : 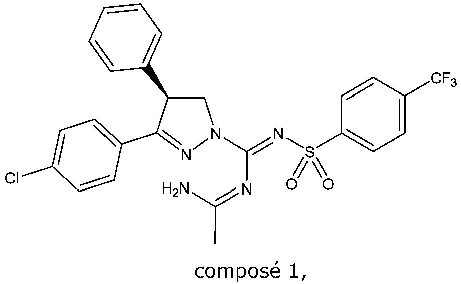 ou 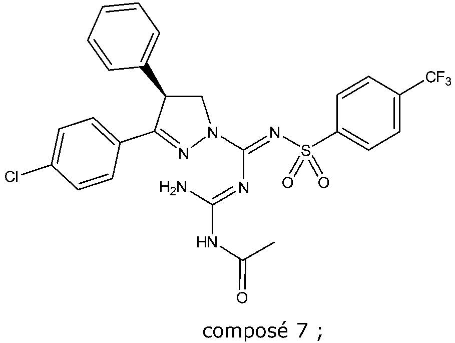
ou un tautomère ou un sel pharmaceutiquement acceptable de ceux-ci ;
dans laquelle le composé se présente sous une forme sensiblement amorphe ;
dans laquelle le rapport pondéral polymère:composé est dans la plage de 1:1 à 6:1, ou de préférence de 1:1 à 4:1, ou de préférence de 2:1 à 5:1, ou de préférence de 3:1 à 5:1, ou de préférence de 1:1 à 3:1, et
dans laquelle la concentration de composé dans la dispersion solide est dans la plage de 15 % à 60 % en poids, de préférence de 18 % à 40 % en poids, ou de préférence dans la plage de 20 % à 40 % en poids, ou de préférence dans la plage de 30 % à 50 % en poids.

2. Dispersion solide selon la revendication 1, ayant un rapport pondéral polymère:composé dans la plage de 1:1 à 4:1, ou de 2:1 à 5:1, ou de 3:1 à 5:1, ou de 1:1 à 3:1.

3. Dispersion solide selon la revendication 1 ou la revendication 2, dans laquelle la concentration de composé dans la dispersion solide est dans la plage de 18 % à 40 % en poids, ou dans la plage de 20 % à 40 % en poids, ou dans la plage de 30 % à 50 % en poids.

4. Dispersion solide selon l'une quelconque des revendications 1 à 3, dans laquelle ledit polymère a une température de transition vitreuse d'au moins 80 °C, ou d'au moins 100 °C, ou d'au moins 120 °C, ou d'au moins 140 °C, ou entre 50 °C et 200 °C, ou entre 80 °C et 200 °C, ou entre 100 °C et 180 °C.

5. Dispersion solide selon l'une quelconque des revendications 1 à 4, dans laquelle ledit polymère est :
- une polyvinylpyrrolidone ou un copolymère de celle-ci ; ou
- de la cellulose ou un dérivé de cellulose, de préférence un dérivé de cellulose qui est une hydroxyalkylméthylcellulose estérifiée ; ou
- un polyéthylène glycol, le polyacide lactique ou le polyméthacrylate.

6. Dispersion solide selon l'une quelconque des revendications 1 à 5, dans laquelle ledit polymère est de la polyvinylpyrrolidone ayant un poids moléculaire moyen compris entre 5 000 et 100 000, ou est l'acétate succinate d'hydroxypropylméthylcellulose.

7. Dispersion solide selon l'une quelconque des revendications 1 à 6, comprenant en outre un tensioactif pharmaceutiquement acceptable, dans laquelle ledit tensioactif pharmaceutiquement acceptable comprend au moins un tensioactif choisi parmi :
- des sels sulfate d'alkyle ou d'alcényle à chaîne longue ;
- des sels sulfonate d'alkyle ;
- des esters d'acide carboxylique à chaîne longue de sorbitane ;
- des esters d'acide carboxylique à chaîne longue de sorbitane pégylés ;
- des copolymères séquencés de polyéthylène-polypropylène glycol ;
- de l'acide carboxylique de mono-, di- et triglycéride à chaîne longue, pégylé ou non pégylé ;
- des alcoxylates de polyéthylène et/ou de polypropylène glycol ;
- des alcoxylates d'alkylphénols ;
- des dérivés d'alkylphénol de polyéthylène et/ou de polypropylène glycols ; et
- des esters d'acide carboxylique à longue chaîne de saccharose.

8. Dispersion solide selon la revendication 7, dans laquelle le tensioactif pharmaceutiquement acceptable est un sulfate d'alkyle à chaîne longue, de préférence du laurylsulfate de sodium.

9. Dispersion solide selon la revendication 7 ou la revendication 8, ayant un rapport pondéral polymère:tensioactif dans la plage de 5:1 à 20:1, ou de 10:1 à 15:1.

10. Dispersion solide selon l'une quelconque des revendications 1 à 9, sous forme de poudre ou sous forme particulaire.

11. Procédé de préparation d'une dispersion solide telle que définie dans l'une quelconque des revendications 1 à 10, comprenant une étape consistant à mélanger le composé et le polymère, ledit mélange comprenant de préférence les étapes consistant à :
(a) dissoudre le composé et le polymère dans un solvant ;
(b) sécher le mélange obtenu en (a).

12. Procédé selon la revendication 11, dans lequel ledit séchage est effectué par séchage par pulvérisation, le mélange étant de préférence effectué par mélange acoustique rapide, extrusion, mélange planétaire et broyage à boulets.

13. Composition pharmaceutique orale solide comprenant une dispersion solide telle que définie dans l'une quelconque des revendications 1 à 10.

14. Composition pharmaceutique orale solide selon la revendication 13, comprenant en outre un support, diluant ou excipient pharmaceutiquement acceptable.

15. Composition pharmaceutique orale solide selon la revendication 14, dans laquelle ledit support, diluant ou excipient est un liant choisi parmi les substances à base de cellulose telles que la cellulose microcristalline et la carboxyméthylcellulose, et d'autres liants tels que la gomme arabique, la gélatine, l'amidon de maïs, la gomme adragante, l'alginate de sodium, le lactose, le sorbitol, le mannitol, le dextrose, le kaolin, la cellulose, le carbonate de calcium, le silicate de calcium, le phosphate dicalcique, et le polyéthylène glycol (PEG), ou une combinaison de ceux-ci, ledit liant étant de préférence la cellulose microcristalline.

16. Composition pharmaceutique orale solide selon la revendication 14, dans laquelle ledit support, diluant ou excipient est un diluant choisi parmi le lactose, le sorbitol, le mannitol, le dextrose, le kaolin, la cellulose, le carbonate de calcium, le silicate de calcium et le phosphate dicalcique.

17. Composition pharmaceutique orale solide selon l'une quelconque des revendications 13 ou 24, dans laquelle le composé est présent dans ladite composition à une concentration comprise entre 5 % en poids et 50 % en poids, ou entre 10 % en poids et 40 % en poids.

18. Composition pharmaceutique orale solide selon l'une quelconque des revendications 13 à 17, sous la forme d'un comprimé ou d'une gélule.

19. Composition pharmaceutique orale solide selon l'une quelconque des revendications 13 à 18, sous forme posologique unitaire comprenant le composé en une quantité dans la plage de 20 à 200 mg par dose.

20. Dispersion solide telle que définie dans l'une quelconque des revendications 1 à 10 ou composition pharmaceutique orale solide telle que définie dans l'une quelconque des revendications 13 à 19 pour une utilisation dans le traitement d'une maladie ou d'un trouble choisi parmi le diabète (de type 1 ou 2), l'obésité, la stéatose hépatique non alcoolique et alcoolique (un facteur de risque d'insulinorésistance), une comorbidité de l'obésité, une comorbidité du diabète, le syndrome de Prader-Willi (PWS), l'obésité par déficit en pro-opiomélanocortine (POMC), l'obésité par déficit en LepR, l'obésité par déficit hétérozygote en POMC, les troubles épigénétiques du POMC, le syndrome de Bardet-Biedl, le syndrome d'Alström, la dyslipidémie prédisposant à une cardiopathie artérioscléreuse, la néphropathie diabétique, la fibrose et les maladies fibrosantes telles que la fibrose pulmonaire idiopathique (IPF) et la fibrose pulmonaire du syndrome de Hermansky-Pudlak (HPS-PF), et la goutte.

21. Dispersion solide ou composition pharmaceutique orale solide pour une utilisation selon la revendication 20, dans laquelle la comorbidité de l'obésité est choisie parmi le syndrome métabolique, la démence, une cardiopathie, l'hypertension, une maladie de la vésicule biliaire, les troubles gastro-intestinaux, les irrégularités menstruelles, l'arthrite dégénérative, l'ulcère de stase veineuse, le syndrome d'hypoventilation pulmonaire, l'apnée du sommeil, les ronflements, une maladie coronarienne, une maladie artérioscléreuse, une pseudotumeur cérébrale, l'arthrose, l'hypercholestérolémie, et l'augmentation de l'incidence des malignités du foie, des ovaires, du col de l'utérus, de l'utérus, des seins, de la prostate, ou de la vésicule biliaire.

22. Dispersion solide ou composition pharmaceutique orale solide pour une utilisation selon la revendication 20, dans laquelle la comorbidité du diabète est choisie parmi la néphropathie diabétique, une maladie rénale chronique, la rétinopathie diabétique, et la neuropathie périphérique et autonome.
